(19) **Europäisches Patentamt**

**European Patent Office**

**Office européen des brevets**

(11) **EP 0 584 287 B1**

(12) **EUROPEAN PATENT SPECIFICATION**

(45) Date of publication and mention
of the grant of the patent:
**30.08.2000 Bulletin 2000/35**

(51) Int. Cl.$^7$: **A61K 38/17**, C07K 1/16

(21) Application number: **92917403.5**

(86) International application number:
**PCT/US92/03041**

(22) Date of filing: **13.04.1992**

(87) International publication number:
**WO 92/18152 (29.10.1992 Gazette 1992/27)**

(54) **PURIFICATION OF MÜLLERIAN INHIBITING SUBSTANCE FOR TREATMENT OF CERTAIN TUMORS**

REINIGUNG EINER DEN MÜLLER-MISCHTUMOR INHIBIERENDEN SUBSTANZ UND BEHANDLUNG VON EINIGEN TUMOREN

PURIFICATION DE L'HORMONE ANTIMULLERIENNE POUR LE TRAITEMENT DE CERTAINES TUMEURS

(84) Designated Contracting States:
**AT BE CH DE DK ES FR GB GR IT LI LU MC NL SE**

(30) Priority: **12.04.1991 US 683966**

(43) Date of publication of application:
**02.03.1994 Bulletin 1994/09**

(73) Proprietor:
**THE GENERAL HOSPITAL CORPORATION
Boston, MA 02114 (US)**

(72) Inventors:
• **DONAHOE, Patricia, K.
Weston, MA 02193 (US)**
• **CHIN, Taiwai
Taipei (CN)**
• **PARRY, Robert, L.
Wellerley, MA 02181 (US)**
• **EPSTEIN, James
Boston, MA 02114 (US)**
• **RAGIN, Richard, C.
Brighton, MA 02135 (US)**
• **MacLAUGHLIN, David, T.
Saugus, MA 01906 (US)**

(74) Representative:
**Sheard, Andrew Gregory et al
Kilburn & Strode
20 Red Lion Street
London WC1R 4PJ (GB)**

(56) References cited:
WO-A-92/18145     US-A- 4 487 833
US-A- 4 510 131     US-A- 4 760 156

• **CELL vol. 21, no. 3 , October 1980 , CAMBRIDGE pages 909 - 915 BUDZIK G.P. ET AL. 'Enhanced Purification of Mullerian inhibiting Substance by Lectin Affinity chromatography'**
• **HYBRIDOMA vol. 3, no. 3 , 1984 , NEW YORK pages 201 - 214 SHIMA H. ET AL. 'Production of Monoclonal Antibodies for Affinity Purification of Bovine Mullerian Inhibiting Substance Activity'**
• **Cancer Research, Volume 52, issued 01 March 1992, R.L. PARRY et al., "Recombinant Human Mullerian Substance Inhibits Human Ocular Melanoma Cell Lines in vitro and in vivo", pages 1182-1186, see entire document.**
• **Cancer Research, Volume 51, issued 15 April 1991, T. CHIN et al., "Human Mullerian Inhibiting Substance Inhibits Tumor Growth in vitro and in vivo", pages 2101-2106, see entire document.**
• **Journal of Cell Biology, Volume 115, No. 3, Part 2, issued November 1991, P.L. HUDSON et al., "Mullerian Inhibiting Substance (MIS) Slows the Cell Cycle Progression of Human Epidermoid Carcinoma A431 Cells", page 276a, Abstract 1599.**
• **Journal of Cellular Biochemistry, Supplement 16B, issued January 1992, P.L. HUDSON et al., "Mullerian Inhibiting Substance (MIS) Slows the Cell Cycle Progression of Human Epidermoid Carcinoma A431 Cells", page 123, Abstract G112.**

EP 0 584 287 B1

Printed by Xerox (UK) Business Services
2.16.7 (HRS)/3.6

**(Cont. next page)**

- Journal of Cellular Biochemistry, Supplement 12A, issued January 1988, P.K. DONAHOE et al., "Mullerian Inhibiting Substance: A Tyrosine Kinase Inhibitor with Anticancer Effects", page 183, Abstract D006.
- Cell, Volume 45, issued 06 June 1986, R.L. CATE et al., "Isolation of the Bovine and Human Genes for Mullerian Inhibiting Substance and Expression of the Human Gene in Animal Cells", pages 685-698, see entire document.
- Journal of Biological Chemistry, Volume 263, No. 35, issued 15 December 1988, R.B. PEPINSKY et al., "Proteolytic Processing of Mullerian Inhibiting Substance Produces a Transforming Growth Factor-beta-like Fragment", pages 18961-18964, see entire document.
- Cancer Research, Volume 49, issued 15 April 1989, J.W. WALLEN et al., "Minimal Antiproliferative Effect of Recombinant Mullerian Inhibiting Substance on Gyneacological Tumor Cell Lines and Tumor Explants", pages 2005-2011, see entire document.

**Description**

FIELD OF THE INVENTION

This application concerns the treatment of certain tumors using an effective amount of the glycoprotein Müllerian Inhibiting Substance. In particular, this application concerns the treatment of vulvar epidermoid carcinomas, cervical carcinomas, endometrial adenocarcinomas, ovarian adenocarcinomas, and ocular melanomas. Pharmaceutical compositions which can be used in such treatment are also disclosed.

BACKGROUND OF THE INVENTION

Müllerian Inhibiting Substance (MIS) is produced by the fetal testis as a 140 kDa glycosylated disulfide-linked homodimer that causes regression of the Müllerian duct in the male fetus. Under reducing conditions, the protein migrates on gel electrophoresis at an apparent molecular weight of 70 kDa. The protein can be proteolytically cleaved by exogenous plasmin into two distinct fragments that migrate electrophoretically as 57 kDa and 12.5 kDa moieties with cleavage at residue 427 of the intact 535 amino acid monomer (Pepinsky, R.B., et al., J. Biol. Chem. 263:18961-4 (1988)).

Various methods for purifying MIS are known. For example, U.S. Patent No. 5,011,687, filed October 19, 1985, now allowed, and entitled "Purified Müllerian Inhibiting Substance and Process for Treating Human Ovarian Cancer Cells," describes a process for purifying MIS from testes by using aqueous polar dissociative solutions, separation of DNA and RNA, fractionation by gel filtration chromatographic elution, and isolation of the MIS. U.S. Patent No. 4,404,188, filed July 29, 1981 and entitled "Purified Müllerian Inhibiting Substance and Method of Purification" describes a process for purifying MIS which comprises treatment with a protein inhibitor, chromatography on ion exchange, chromatography on wheat germ lectin, on concanavalin A and/or on a supported triazinyl dye. U.S. Patent No. 4,487,833, filed on March 1, 1982 and entitled "Method of Preparing Hybridomas and of Purifying Immunogenic Materials" describes a process for separating MIS using immunoaffinity chromatography. MIS may also be obtained from recombinant DNA techniques (Cate, R.L. et al., Cell 45: 685-698 (1986)).

In the female fetus, the Müllerian duct develops into the Fallopian tubes, uterus and upper vagina. It is known that MIS causes regression of the Müllerian duct in the male fetus. MIS has also been shown to play a role in inhibition of oocyte meiosis (Takahashi, M., et al., Mol. Cell. Endocrinol. 47:225-234 (1986)), testicular descent (Hudson, J.M., et al., Endocr. Rev. 7:270-283 (1986)), inhibition of fetal lung development (Catlin, E., et al., Am. J. Obstet. Gynecol. 159:1299-1303 (1988)), inhibition of autophosphorylation of the EGF receptor (Coughlin, J.P., et al., Mol. Cell. Endocrinol. 49:75-86 (1987); Cigarroa, F.G., et al., Growth Factors 1:179-191 (1989)) and inhibition of tumor growth (Donahoe, P.K., et al., Science 205:913-915 (1979); Donahoe, P.K., et al., Ann. Surg. 194:472-480 (1981); Fuller, Jr., A.F., et al., J. Clin. Endocrinol. Metab. 54:1051-1055 1982); Fuller, Jr., A.F., et al., Gynecol. Oncol. 22:135-148 (1985); U.S. Patent No. 4,404,188, Donahoe, P.K., et al., filed July 29, 1981).

The antitumor effect of MIS demonstrated in U.S. Patent No. 5,011,687 was elicited using natural MIS extracted and partially purified from bovine testes. Since that time, the bovine and human MIS genes have been cloned and the human protein expressed in Chinese hamster ovary (CHO) cells. Initial antiproliferative studies against established cell lines involved using a highly purified recombinant human MIS (rhMIS), but these studies suggested that the antiproliferative effect of rhMIS on human gynecological tumor cells is limited to ovarian cancers. (Wallen, J.W., et al., Cancer Res. 49:2005-2011 (1989)).

SUMMARY OF THE INVENTION

In view of the previously reported inhibition of tumor growth caused by bovine MIS, the present inventors speculated that a highly purified form of MIS might be effective in treating tumors of generally similar origin. Although a relatively highly purified form of MIS had been previously achieved, little success was demonstrated with respect to tumor treatment (Wallen *et al*, *Cancer Res*. **49** 2005-2001 (1989)). Accordingly, it was desirable to further develop a form of MIS which would be effective in inhibiting tumor growth.

This goal has been achieved by the present invention which provides for a method of inhibiting tumor growth comprising administering an effective amount of Müllerian Inhibiting Substance to a patient, said tumor selected from the group consisting of vulvar epidermoid carcinoma, cervical carcinoma, endometrial adenocarcinoma, ovarian adenocarcinoma, and ocular melanoma.

Another goal of this invention is to reduce the concentration of chemotherapeutic agents which are currently used in the treatment of the tumors of this invention. This goal has also been achieved by providing for a method of inhibiting tumor growth comprising administering an effective amount of a combination of a chemotherapeutic agent and Müllerian Inhibiting Substance to a patient, said tumor selected from the group consisting of vulvar epidermoid carcinoma, cervical carcinoma, endometrial adenocarcinoma, ovarian adenocarcinoma, and ocular melanoma.

This invention further provides for a pharmaceutical composition comprising an effective tumor inhibiting amount of proteolytically cleaved Müllerian Inhibiting Substance, said tumor selected from the group consisting of vulvar epidermoid carcinoma, cervical carcinoma, endometrial adenocarcinoma, ovarian adenocarcinoma, and ocular melanoma, and a pharmaceutically acceptable carrier.

BRIEF DESCRIPTION OF THE DRAWINGS

The present invention will be better understood by reference to the Description of the Preferred Embodiments when taken together with the attached drawings, wherein:

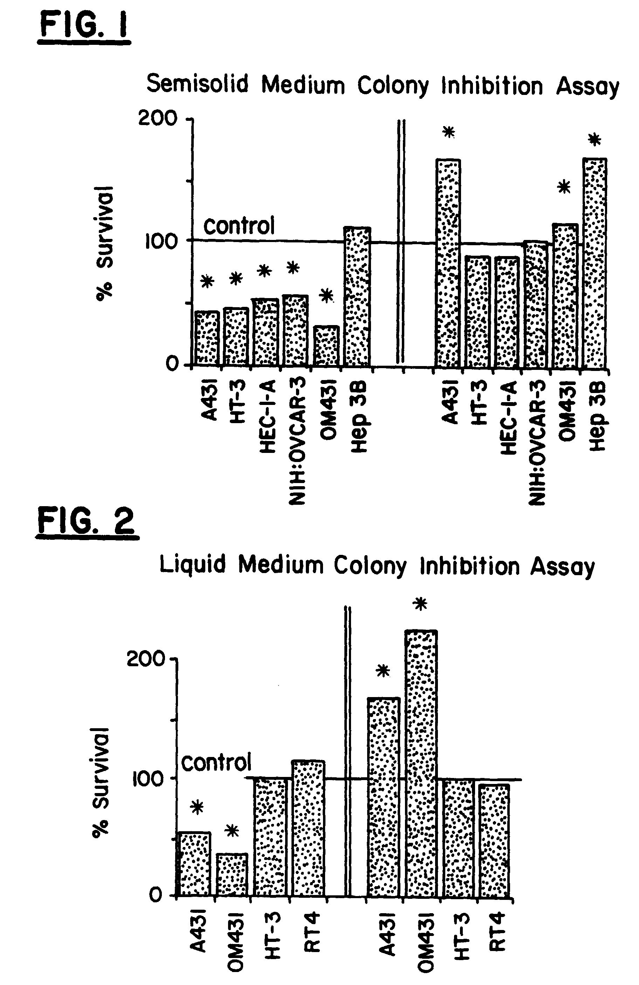

FIG. 1 shows the results of a semisolid medium (double layer) colony inhibition assay. The left side of the figure shows the effects of the immunoaffinity chromatography method described herein (IAP-MIS) in which colony formation of A431, HT-3, HEC-I-A, NIH:OVCAR-3, and OM431 cells was significantly inhibited (30 nM). The right side of Fig. 1 shows the effect of the salt fraction pre-eluted from the immunoaffinity column (IAP-salt). Stimulation of A431, OM431 and Hep 3B colony formation was seen when treated with the salt eluted fraction. The symbol "*" indicates $p < 0.05$ when compared with control.

FIG. 2 shows the results of a liquid medium colony inhibition assay. The left side of the figure shows the effect of IAP-MIS in which colony formation of A431 and OM431 cells was significantly inhibited (30 nM). The right side of the figure shows the effect of the salt fraction preeluted from the immunoaffinity column (IAP-salt). The colony formation of A431 and OM431 cells was stimulated by IAP-salt. IAP-MIS and IAP-salt showed no effect on HT-3 and RT4 cells. The symbol "*" indicates $p < 0.05$ when compared with control.

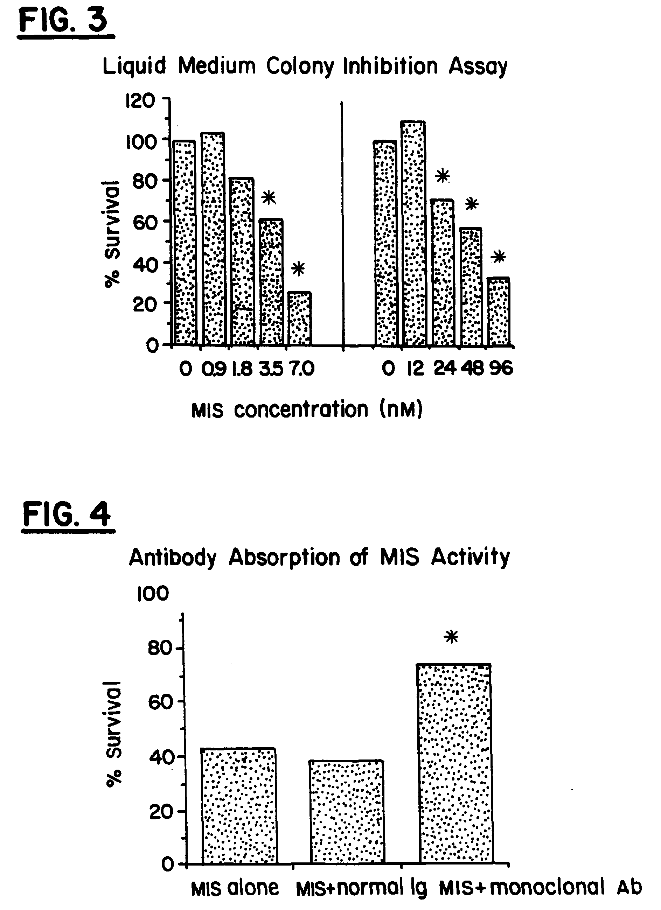

FIG. 3 shows dose dependent inhibition of A431 colony formation by MIS. The left side of the figure shows where MIS purified by dye affinity chromatography (DG-MIS) was tested in increasing doses using the liquid medium colony inhibition assay. Significant inhibition was seen at MIS concentrations of 3.5 and 7.0 nM. The right side of the figure shows that IAP-MIS caused significant inhibition at concentrations of 24, 48 and 96 nM. The symbol "*" indicates $p < 0.05$ when compared with controls.

FIG. 4 shows antibody absorption of DG-MIS. The inhibitory effect of MIS of this degree of purity on A431 colony formation was significantly reduced after absorption with the MIS specific monoclonal antibody (6E11), but not after nonspecific absorption by normal IgG. The symbol "*" indicates $p < 0.05$ when compared with MIS alone.

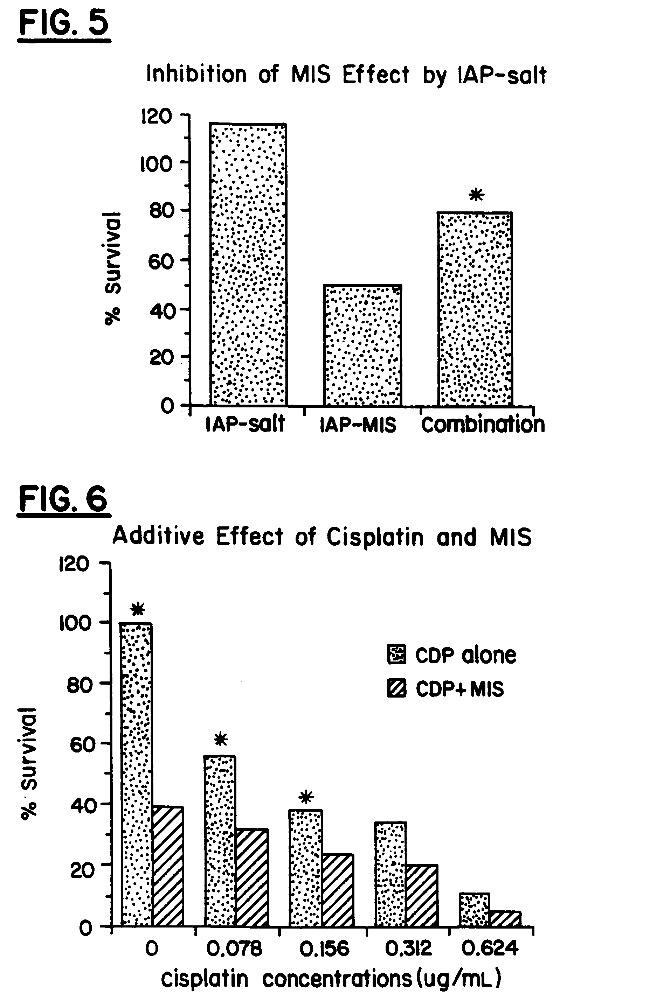

FIG. 5 shows the effects of various immunopurified fractions on A431 cells in the liquid colony inhibition assay. The IAP-MIS (50 nM), after salt and acid elution, inhibited colony growth, whereas the salt eluted fraction stimulated growth. When recombined, of these two preparations reduces MIS inhibitory effect. The percent survival of the combination was significantly higher than that of MIS alone. The symbol "*" indicates $p < 0.05$ when compared with IAP-MIS alone, although the MIS concentrations were the same.

FIG. 6 shows the additive effect of cisplatin and DG-MIS. When cisplatin and MIS were tested on A431 cells in the liquid medium colony inhibition assay, their effects were additive at cisplatin concentrations of 0.078 and 0.156 mg/ml. The symbol "*" indicates $p < 0.05$ when cisplatin plus MIS was compared with cisplatin alone.

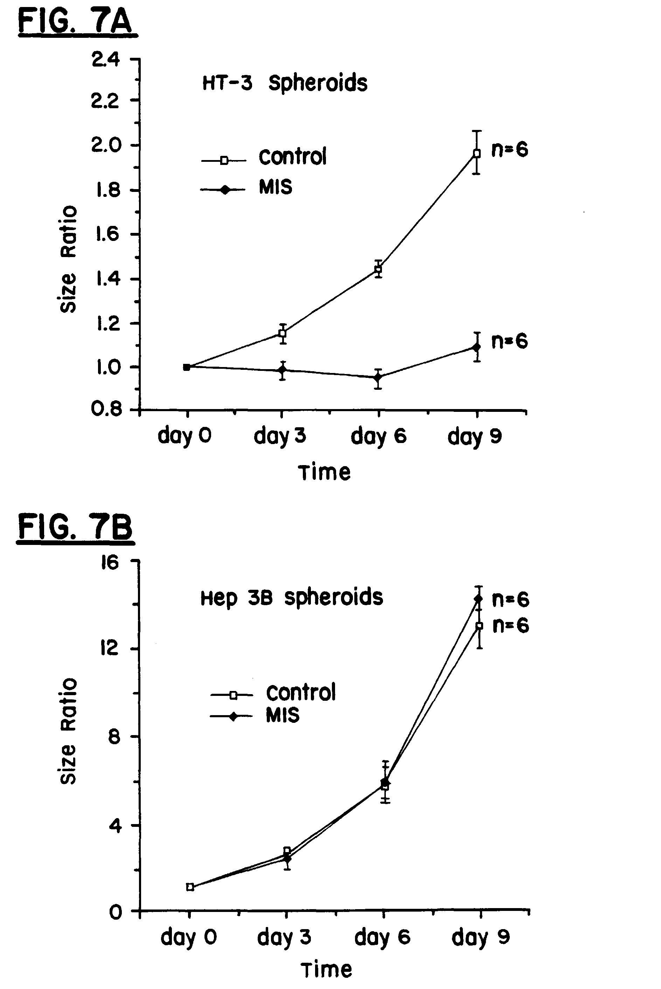

FIG. 7A shows the results of a spheroid assay. DG-MIS (7 nM) inhibited the growth of HT-3 spheroids when compared with control ($p < 0.05$).

FIG. 7B shows the results of a spheroid assay. DG-MIS (7 nM) did not effect the growth of Hep 3B spheroids when compared with control ($p < 0.05$).

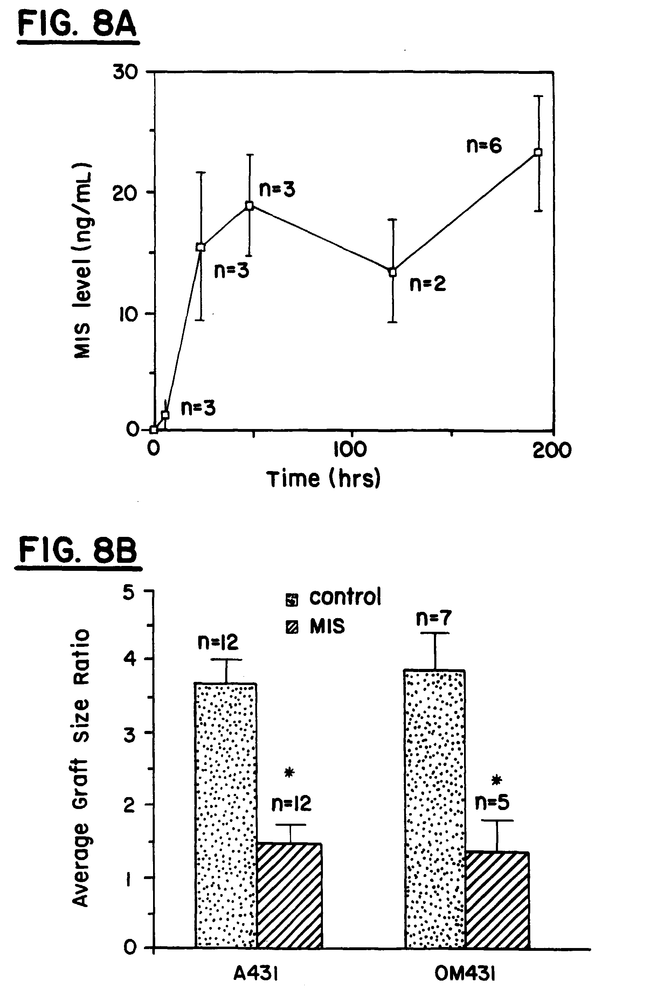

FIG. 8A shows MIS serum levels. Alzet pumps loaded with 33 µg MIS were implanted into CD-1 mice. A relatively constant level of MIS was achieved 24 hours after implantation.

FIG. 8B shows inhibition of tumor growth <u>in vivo</u> by MIS. A431 and OM431 cells were implanted in the subrenal capsule space of CD-1 mice. The graft size ratios of both tumors were significantly inhibited in the MIS treated group. The symbol "*" indicates $p < 0.05$ when compared with controls.

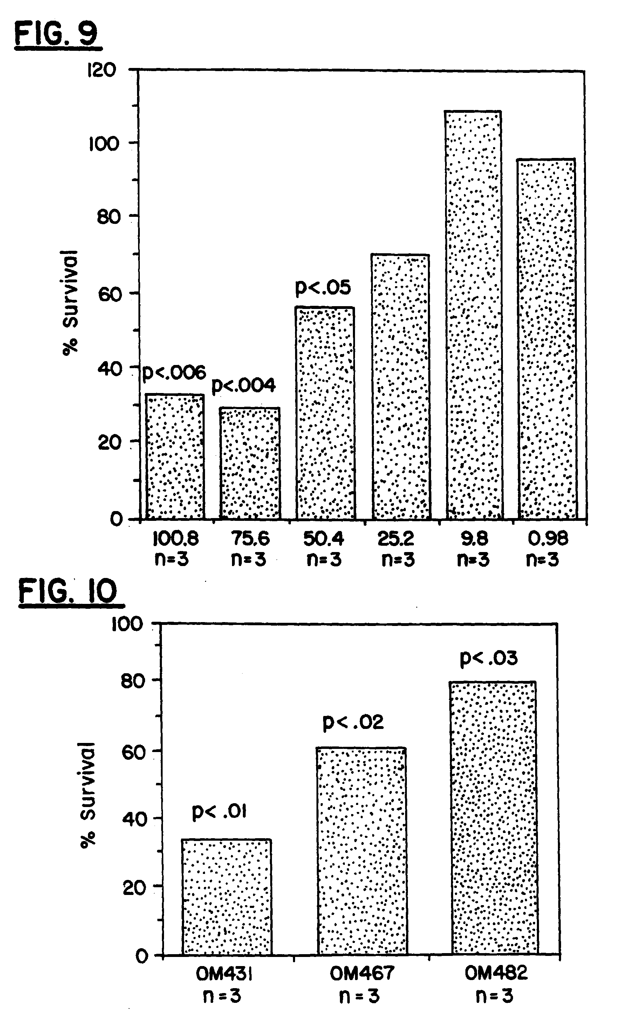

FIG. 9 shows dose dependent inhibition of OM431 colony formation by MIS in a liquid colony inhibition assay. The percent survival was 33%, 29.5%, 56.6%, 71%, 109.8%, and 96.8%, respectively, for MIS concentrations of 100.8, 75.6, 50.4, 25.2, 9.8, and 0.98 nM. Significant inhibitions were seen with MIS concentrations of 50.4 ($p < .05$), 75.6 ($p < .004$), and 100.8 ($p < .006$) nM MIS.

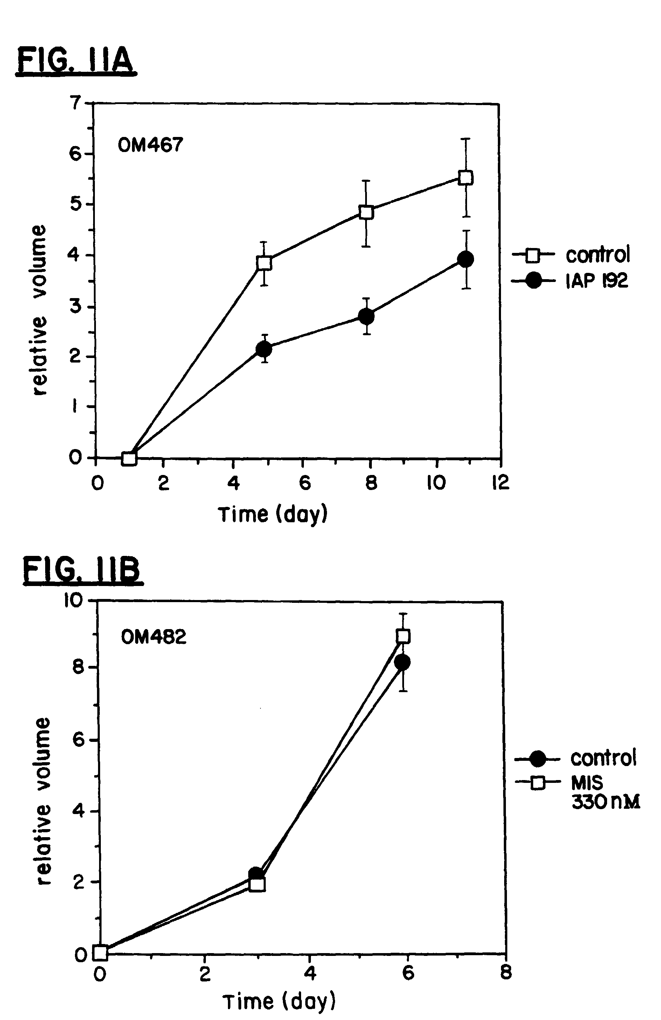

FIG. 10 shows the results of a semisolid medium (double layer) colony inhibition assay. All cell lines were significantly inhibited by rhMIS. The percent survival of the various cell lines was 34% for OM431 (in 30 nM MIS) ($p < .01$), 61% for OM467 (in 150 nM MIS) ($p < .02$), and 80% for OM482 (in 90 nM MIS) ($p < .03$).

FIG. 11A shows the results of a multicellular spheroid assay of OM467 cells. The average graft size ratios of OM467 spheroids in the control group (n=6) were $3.85 \pm 0.43$, $4.85 \pm 0.64$ and $5.52 \pm 0.78$ at day 5, 8 and 11, respectively. The average graft size ratios in the MIS group (n=6) were $2.17 \pm 0.26$, $2.79 \pm 0.33$ and $3.91 \pm 0.59$, respectively. This difference was significant ($p < .02$).

FIG. 11B shows the results of a multicellular spheroid assay of OM482 cells. The average graft size ratios of OM482 spheroids in the control group (n=10) were 2.18±0.17 and 8.22±0.81, respectively, at day 3 and 6. The average graft size ratios in the MIS group (n=10) were 1.93±0.14 and 9.0±0.67, respectively.

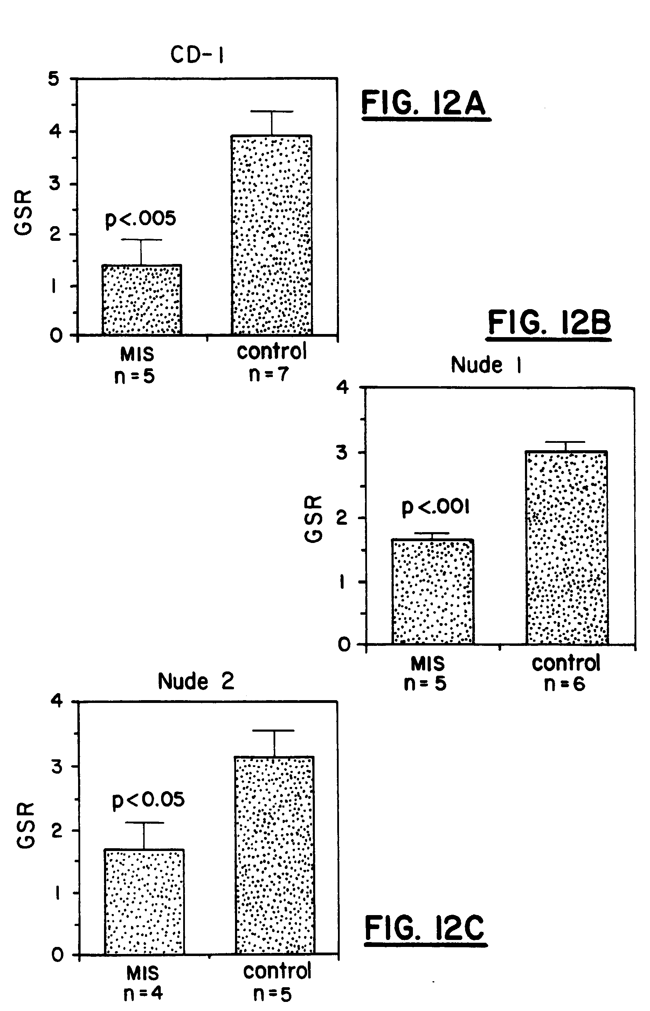

FIG. 12A shows the results of a subrenal capsule assay of CD-1 irradiated mice. The graft size ratio of the OM431 tumor was 3.93±0.49 in the control (n=7) compared to 1.42±0.44 in the MIS group (n=5). Each MIS treated mouse received 48.6 micrograms of MIS over the eight day assay. The growth of the tumors in each MIS group was significantly lower than the control (p<0.005).

FIG. 12B shows the results of a subrenal capsule assay using nude mice. The graft size ratios of the OM431 tumors were significantly greater for the controls (3.02±0.17) compared to the MIS group (1.68±0.09). The MIS treated group received 44.7 micrograms MIS over eight days. The average MIS serum levels of the MIS treated mice on the eighth day of the nude mouse assays were 749 pM. The measured controls had MIS levels of less than 10 pM. The growth of the tumors in each MIS group was significantly lover than the control (p<0.001).

FIG. 12C shows the results of a subrenal capsule assay using nude mice. The graft size ratios of the OM431 tumors were significantly greater for the controls (3.14±0.40) compared to the MIS group (1.69±0.43). The MIS treated group received 130 micrograms MIS over eight days. The average MIS serum levels of the MIS treated mice on the eighth day of the nude mouse assay were 570 pM. The measured controls had MIS levels of less than 10 pM. The growth of the tumors in each MIS group was significantly lower than the control (p<0.05).

## DESCRIPTION OF THE PREFERRED EMBODIMENTS

The term "Müllerian Inhibiting Substance" (interchangeably referred to as "MIS") is intended to include compounds and materials which are structurally similar to MIS. Examples of such included substances and materials are salts, derivatives, and aglycone forms of MIS. Additionally, the present invention is intended to include mutant forms of MIS which have substantially the same biological activity as MIS. Examples of such mutant forms would be MIS molecules carrying a deletion, insertion, or alteration in amino acid sequence. MIS may be obtained from any mammalian source or, as indicated above, from non-mammalian sources through the use of recombinant DNA technology, or from the chemical synthesis of the MIS protein.

A gene is said to be a "recombinant" gene if it results from the application of Recombinant DNA Techniques. Examples of recombinant DNA techniques include cloning, mutagenesis, transformation, etc. Recombinant DNA Techniques are disclosed in Maniatis, T., et al., Molecular Cloning: A Laboratory Manual, Cold Spring Harbor, N.Y. (1982).

Using the methods of the invention for purifying MIS, a pharmaceutical preparation for treating the tumors of this invention can be prepared. In order to achieve a highly purified MIS which is substantially free from proteolytic enzymes or inhibition of MIS antiproliferative activity, it is preferred to use a method which takes advantage of the specificity of antigen-antibody interactions to recover a product having a substantially pure MIS product. This method, which forms an aspect of the present invention, incorporates the use of immunoaffinity chromatography, but improves upon previously used methods in that the recovered MIS product is substantially free of contaminating enzymes having MIS proteolytic activity or inhibitors of MIS antiproliferative activity.

The immunoaffinity chromatography method used herein effectively eliminates contaminating enzymes having MIS proteolytic activity or inhibitors of MIS antiproliferation activity from an immunoaffinity chromatography matrix by eluting with an effective amount of an alkali metal halide or an alkaline earth metal halide. The MIS is then recovered by eluting with an acid solution having a pH of between about 2.5 and 4.0. This end product is referred to herein as IAP-MIS.

The IAP-MIS can be obtained in solution at up to 95% purity. While the percent purity is comparable to other immunoaffinity purifications processes, the IAP-MIS is substantially free of contaminating proteolytic enzymes or inhibitors of MIS antiproliferative activity.

The purified MIS of this invention can be obtained in solution at up to 95% purity or greater. While the percent purity is comparable to other immunoaffinity purification processes, the MIS composition of this invention is substantially free of contaminating proteolytic enzymes or inhibitors of MIS antiproliferative activity.

For purposes of this invention, purified MIS is considered to be a MIS composition which is substantially free of contaminating proteolytic enzymes or inhibitors of MIS antiproliferative activity regardless of percent purity. The composition is considered to be substantially free of proteolytic enzymes if gel electrophoresis of the purified MIS product indicates a protein having a molecular weight of 140 kDa or 70 kDa. Gel electrophoresis of such a product will not show time dependent proteolytic fragments which are degradation products of MIS. For example the 57 kDa, 12.5 kDa, 34 kDa and 22 kDa degradation fragments of MIS further described herein will not be readily discernable by standard gel electrophoresis methods.

The MIS composition will be considered to be substantially free of inhibitors of MIS antiproliferative activity if the MIS blocks proliferation of tumor cells. Examples of such tumor cells are included herein. 0609.3060000). These examples include tumors selected from the group consisting of vulvar epidermoid carcinoma, endometrical adenocarcenoma, cervical carcenoma, endometrial adenocacenoma, ovarian adenoracenoma, and other ocular melanoma. The determination of antiproliferative activity of these cells can be achieved by any of the procedures described herein.

In order to obtain MIS which is substantially free of proteolytic enzymes or inhibitors of MIS antiproliferative activity, the contaminants are separated from the MIS using immunoaffinity chromatography. Separation occurs by eluting the enzymes or inhibitors with an alkali metal halide or an alkaline earth metal halide. Such a compound will generally be in solution and an effective amount of halide will be between about 0.1 M and 2.0 M. As alkali metals, the ions of lithium, sodium and potassium are preferred with sodium being the most preferable. As alkaline earth metals, the ions of magnesium and calcium are preferred. As halides, the ions of fluorine, chlorine, bromine and iodine are preferred with chlorine being most preferable. When eluting with sodium chloride, a solution of between about 0.1 and 2.0 M is preferred. The concentration of halide can also be varied as elution progresses if desired. This can be accomplished by increasing molar concentration of halide in a stepwise fashion. It is preferred that each step be altered after about 0.1-2.0 bed volumes of solution have contacted the chromatography matrix, although such steps can be further modified as desired.

The halide can also be accompanied in solution with an effective amount of a chelating agent. These agents are capable of binding metal ions which can inactivate enzymes that require the metal ions for activity. Such agents include the compounds ethylenediamine tetraacetate (EDTA), and ethylenebis(oxyethylenenitrilo)tetraacetic acid (EGTA). Chelants can be effectively added in a range of between 0.1 and 50 mM.

After the contaminating proteolytic enzymes or inhibitors of MIS antiproliferative activity have been separated, the MIS can be recovered by eluting with an acid solution having a pH of between about 2.0 and 4.0. Although dilutions of strong acids such as HCl can be used, organic acids are preferred because of their relatively mild acid strength. For example, the use of acid amines and imines can be employed as well as monocarboxylic, dicarboxylic and tricarboxylic acids. Preferred as monocarboxylic acids are acetic, propionic and butyric acid. Preferred as dicarboxylic acids are succinic, fumaric and malic acid. Preferred as a tricarboxylic acid is citric acid. Preferred among the amines are the acidic amino acids such as aspartic and glutamic acid. The pH of the acid solution can also be incrementally varied as in the application of the halide.

After the purified MIS product has been eluted, it is preferable to neutralize the product to a pH of between 6.8 and 7.6 to guard against acid hydrolysis. This can be accomplished by various hydroxide compounds such as NaOH or NH$_4$OH or by various buffers which can quickly achieve neutralization of the product in the desired pH range. These hydroxide compounds are not to be considered as all inclusive as those of ordinary skill in the art will appreciate.

Although this specification describes a pharmaceutical composition which comprises proteolytically cleaved Müllerian Inhibiting Substance, and method for producing the same it is to be understood that the MIS 140 kDa homodimer or the 70 kDa subunit of MIS can be included in the pharmaceutical composition. In this case, naturally occurring proteolytic enzymes in vivo can proteolytically cleave MIS to its effective form. Such enzymes are represented by the proteolytic compounds described herein.

The term "protein fragment" is meant to include both synthetic and naturally-occurring amino acid sequences derivable from the naturally occurring amino acid sequence of MIS. The protein is said to be "derivable from the naturally-occurring amino acid sequence of MIS" if it can be obtained by fragmenting the naturally-occurring chosen sequence of MIS, or if it can be synthesized based upon a knowledge of the sequence of the naturally occurring amino acid sequence or of the genetic material (DNA or RNA) which encodes this sequence.

The term "proteolytically cleaved" refers to an MIS product obtained by treatment with any substance which is capable of cleaving either the homodimer or the 70 kDa subunit of MIS into a protein fragment which inhibits growth of the tumors of this invention. In general, MIS is effective in treating the tumors of this invention when proteolytically cleaved to form protein fragments of about 57 kDa and 12.5 kDa. Such substances which cleave MIS in this manner include serine proteases, such as plasmin, and endopeptidases. These enzymes are not to be considered as all inclusive or limiting in any manner since other enzymes can also proteolytically cleave MIS and such enzymes can be readily determined by those of ordinary skill in the art.

The invention further pertains to polypeptides that, in addition to the chosen sequence, may contain or lack one or more amino acids that may not be present in the naturally-occurring sequence, wherein such polypeptides are functionally similar to or possess antagonist activity to the chosen polypeptide. Such polypeptides for the present invention, are termed "functional derivatives," provided that they demonstrate activity which is substantially similar to or antagonistic to that of MIS.

Included within the scope of this invention are the use of additional amino acid residues added to enhance coupling to carrier protein or amino acid residues added to enhance the tumorigenic treatment of this invention. The MIS composition may be in the form of the free amines (on the N-terminus), or acid-addition salts thereof. Common acid solution salts are hydrohalic acid salts, i.e., HBr, HI, or more preferably, HCl. Useful cation are alkali or alkaline earth metallic cations (i.e., Na, K, Li, ½Ca, ½Ba, etc.) or amine cations (i.e., tetraalkylammonium, trialkylammonium, where alkyl can be C$_1$-C$_{12}$). As known in the art, the amino acid residues of MIS may be in their protected or unprotected form, using appropriate amino or carboxyl protecting groups.

The pharmaceutical compositions of this invention can be formulated according to known methods to prepare pharmaceutically useful compositions, whereby MIS or its functional derivatives are combined in admixture with a pharmaceutically acceptable carrier vehicle. Suitable vehicles and their formulation, inclusive of other human proteins, i.e., human serum albumin, are described for example in Remington's Pharmaceutical Sciences (16th Ed., A. Oslo, Ed., Mack, Easton, Pa (1980)). In order to from a pharmaceutically acceptable composition suitable for effective administration, such compositions will contain an effective amount of the MIS, or its functional derivatives, together with a suitable amount of carrier vehicle.

An "effective amount" of MIS is one which is sufficient to inhibit growth of the tumors of this invention in a human or animal. According to this invention, inhibition of a tumor implant can be indicated by a decrease in graft size ratio. The graft size ratio is calculated as $(L2 \times W2 \times W2) / (L1 \times W1 \times W1)$, wherein L1 is the longest diameter of the implant, W1 is the diameter perpendicular to L1, L2 is the longest diameter of the tumor, and W2 is the diameter perpendicular to L2. Using this calculation, inhibition is demonstrated when the graft size ratio of a treated specimen is less than the graft size ratio of an untreated control. When assessing inhibition of a naturally occurring tumor in a patient, the volume of the tumor $(L2 \times W2 \times W2)$ before and after treatment need only be compared.

The effective amount may vary depending upon criteria such as the age, weight, physical condition, past medical history, and sensitivity of the recipient. The effective amount will also vary depending on whether administration is oral, intravenous, intramuscular, subcutaneous, local, or by direct application to the tumor. In the case of direct tumor application, it is preferable that a final serum concentration of at least 0.1 nM, preferably about 0.1-1.0 nM, of MIS be achieved. Effective individual dosage through the additionally named means of administration can be readily determined by methods well known to those of ordinary skill in the art. For example, using the size ratio calculation as detailed above, one of ordinary skill in the art can determine optimal dosage levels for any means of administration. In treating a patient, it is preferable to achieve a serum level of at least 10 ng/ml of MIS.

Compositions containing MIS or its functional derivatives may be administered orally, intravenously, intramuscularly, subcutaneously, or locally. Additional pharmaceutical methods nay be employed to control the duration of action. Controlled release preparations may be achieved by the use of polymers to complex or adsorb MIS or its functional derivatives. The controlled delivery may be exercised by selecting appropriate macromolecules (for example polyesters, polyamino acids, polyvinyl pyrrolidone, ethylenevinylacetate, methylcellulose, carboxymethylcellulose, and protamine sulfate) and the concentration of macromolecules as well as the methods of incorporation in order to control release.

Another possible method to control the duration of action by controlled release preparations is to incorporate MIS into particles of a polymeric material such as polyesters, polyamino acids, hydrogels, poly(lactic acid) or ethylene vinyl acetate copolymers. Alternatively, instead of incorporating MIS into these polymeric particles, it is possible to entrap MIS in microcapsules prepared, for example, by coacervation techniques or by interfacial polymerization, for example, hydroxymethylcellulose or gelatin microcapsules and poly(methylmethacrylate) microcapsules, respectively, or in colloidal drug delivery systems, for example, liposomes, albumin microspheres, microemulsions, nanoparticles, and nanocapsules or in macroemulsions. Such teachings are disclosed in Remington's Pharmaceutical Sciences, supra (1980).

Pharmaceutical compositions which include the proteolytically cleaved MIS protein fragments of this invention can also include chemotherapeutic agents which are known to inhibit tumor growth in a human or animal. The chemotherapeutic agent included in this composition can be directed to any specific neoplastic disease. Such agents are described in Goodman and Gilman's The Pharmacological Basis of Therapeutics, 8th Ed., Pergamon Press, New York, N.Y., 1985. It is preferred, however, that the chemotherapeutic agent inhibit growth of the tumors of this invention.

In general, the chemotherapeutic agent which is combined with MIS will have an additive effect on the treatment of the tumors of this invention. This means that the quantity of chemotherapeutic agent used in treating the tumors of this invention can be reduced from the manufacturer's recommended dose, thereby reducing undesirable side effects. For example, for every quantity of chemotherapeutic agent that is reduced in the tumor treatment, an equivalent effective amount of MIS can be added.

It is to be understood that the use of the term "equivalent effective amount" does not necessarily mean an equivalent weight or volume quantity, but represents the quantity of MIS that offers an equal inhibition to tumor growth. This may have to be evaluated on a patient by patient case, but can be determined, for example, by comparing quantities that achieve equal size reduction ratios as defined above. Typically, chemotherapeutic agents which can be combined with MIS for treatment of the tumors of this invention will be effective between about 0.001 and 10.0 mg/kg body weight of the patient. Administration of the combination of MIS and chemotherapeutic agent can be accomplished in the same manner as administration of the MIS alone.

Included as chemotherapeutic agents in the pharmaceutical compositions of this invention are nitrogen mustards such as cyclophosphamide, ifosfamide, and melphalan; ethylenimines and methylmelamines such as hexamethylmelamine and thiotepa; pyrimidine analogs such as fluorouracil and fluorodeoxyuridine; vinca alkaloids such as vinblastine; epipodophyllotoxins such as etoposide and teniposide; antibiotics such as actinomycin D, doxorubicin, bleomycin, and mithramycin; biological response modifiers such as interferon $\alpha$; platinum coordination complexes such as cisplatin and carboplatin; estrogens such as diethylstilbestrol and ethinyl estradiol; antiandrogens such as flutamine; and gonadotropin releasing hormone analogs such as leuprolide. Other compounds such as decarbazine, nitrosoureas, methotrexate, diticene, and procarbazine are also effective. Of course, other chemotherapeutic agents which are known to those of ordinary skill in the art can readily be substituted as this list should not be considered exhaustive or limiting.

Having now generally described this invention, the same will become more readily understood by reference to the following specific examples which are included herein for purposes of illustration only and are not intended to be limiting unless otherwise specified.

The terms "Sepharose" and "Alzet" are acknowledged as registered trade marks.

EXAMPLE 1

I. Materials and methods

A. Production and Purification of rhMIS

After cloning MIS cDNA and genomic DNA, dihydrofolate reductase deficient CHO were cotransfected with a linear transcript of both the human MIS and the dihydrofolate reductase genes according to the method of Cate (Cate, R.L., et al., Cell 45:685-698 (1986)). The transfected CHO cells were amplified in Methotrexate and grown at 37°C in alpha minimal essential medium without ribonucleosides and deoxyribonucleosides, supplemented with 10% bovine MIS-free female fetal calf serum (FCS). Two different MIS purification protocols were used to provide either partially pure or homogeneous MIS. The first employed serial anion exchange and dye affinity chromatography (DG-MIS) according to the method of Budzik (Budzik, G.P., et al., Cell 34:307-314 (1983)) to provide a 1-10% concentration of MIS. The second method employed the immunoaffinity chromatography method (IAP-MIS) detailed herein to provide a 90-95% concentration of MIS.

The immunoaffinity chromatography method used herein was directed toward the recovery of recombinant human MIS (rhMIS). The protein was purified from the conditioned media of Chinese hamster ovary (CHO) cells, transfected with a linear construct of the human rhMIS gene and the DHFR gene, amplified by 30 nM Methotrexate selection, grown to confluence in four liter bioreactors on stainless steel coils as described by Epstein (Epstein et al., In Vitro Cell. and Devel. Biol 25(2):213-6 (1989)) or on modified roller bottles in alpha-Modified Eagle's Medium ($\alpha$-MEM-), supplemented with 5% female fetal calf serum (FFCS), 10 mg/ml Amikacin, 1.3 gm/l l-glutamine, 2.0 gm/l d-glucose, and 0.1 gm/l sodium pyruvate, in the absence of nucleosides. The medium was collected every 3-4 days, and stored at -20°C. Media were thawed and filtered through Whatman #4 filter paper to remove debris, concentrated 20x an a Minitan (Millipore) with a 30 kDa exclusion ultrafilter, and stored at -70°C, until purification. A 5 ml immunoaffinity column was constructed using approximately 50 mg of a Protein A-Sepharose (BioRad) purified monoclonal anti-human rhMIS antibody [6E11] as described by Hudson (Hudson, P.L., et al., J. Clin. Endocrinol. Metab. 70:16-22 (1990)), and covalently attached to Affigel-10 agarose resin (BioRad), per the manufacturer's instructions (approximately 80% coupling efficiency). The column was equilibrated with 100 mls of 20 mM HEPES, pH 7.4, and 200 ml of the concentrated media loaded at 1 column volume/hour at 4°C. After loading, the column was washed with 20 mM HEPES, pH 7.4, until the absorbance at 280 nm returned to baseline (60-100 mls).

Elution of rhMIS bound to this column was achieved using 2.0 M sodium thiocyanate (NaSCN); or 1 M Acetic acid, 20 mM HEPES, pH 3.0, with or without a pre-elution step containing 0.5 M MaCl, 1 mM EDTA, 0.001% NP-40, 20 mM HEPES, pH 7.4. The majority of the rhMIS protein eluted in a single 2 ml fraction, which was immediately neutralized with either NaOH or $NH_4OH$ to a pH between 7.0 and 7.4. Depending on the initial pH of the fraction and technique of neutralization, dilutional effects ranged from 10-50%.

The biological activity of MIS was detected in vitro using the rat Müllerian duct regression organ culture assay of Donahoe (Donahoe, P. K., et al., J. Surg. Res. 23:141-148 (1977)). MIS concentrations were estimated using an enzyme-linked immunosorbent assay (ELISA) for MIS according to the method of Hudson supra. Protein concentrations were measured by the method of Bradford (Bradford, N.M., Anal. Biochem. 72:248-254 (1976)).

B. Preparation of Monoclonal Antibody for MIS Absorption.

Monoclonal antibody was produced by immunizing female A/J mice (Jackson Laboratory, Bar Harbor, ME.) with immunoaffinity purified rhMIS using methods previously described for bovine MIS (Hudson, P.L., et al., J. Clin. Endocrinol. Metab. 70:16-22 (1990); Mudgett-Hunter, M., et al., J. Immunol. 128:1327-33 (1982)). Spleen cells producing anti-MIS antibodies were harvested and hybridomas were produced as described by Kohler (Kohler, G., Immunol. Methods 2:285-98 (1981)). A monoclonal line (6E11) was selected and amplified in Dulbecco's modified essential medium supplemented with 15% FCS. The antibody was precipitated from 6E11 conditioned medium with 50% $(NH_4)_2SO_4$, and further purified by protein A-Sepharose CL-4B (Sigma, St. Louis, MO.) chromatography.

C. Cell Lines.

A431, a cell line derived from a human vulvar epidermoid carcinoma, HT-3 from a human lymph node metastasis of a cervical carcinoma, NIH:OVCAR-3 from a human ovarian adenocarcinoma, HEC- 1-A from a human endometrial adenocarcinoma, RT4 from a human bladder transitional-cell papilloma, and Hep 3B from a human hepatocellular carcinoma were obtained from American Type Culture Collection. OM431, from a human ocular melanoma, was obtained from Dr. James Epstein of Massachusetts General Hospital.

The A431, HT-3, NIH:OVCAR-3, OM431 and Hep 3B cells were maintained with alpha minimal essential medium with ribonucleosides and deoxyribonucleosides (α-MEM+) supplemented with 10% female FCS and 2 mM L-glutamine. HEC-1-A and RT4 were maintained with McCoy's 5A medium supplemented with 10% female FCS. Before study, cells were serially subcultured, then trypsinized at 70-80% confluency to achieve synchrony. This proliferating cell population was then centrifuged at 1500 RPM for 5 minutes and resuspended with 10% female FCS supplemented medium. Cells were counted in a hemocytameter.

D. Semisolid Medium (Double Layer) Colony Inhibition Assay.

The effects of IAP-MIS and the salt eluted fraction from the immunoaffinity column (IAP-salt) were tested using A431, HT-3, HEC-IA, NIH:OVCAR-3, OM431, and Hep 3B cells in the conventional double layer agarose colony inhibition assay of Hamburger (Hamburger, A.W., et al., Science 197:461-463 (1977); Hamburger, A.W., et al., J. Clin. Invest. 60:846-854 (1977)). The underlayer of the 35 mm culture dishes contained 1 ml of 0.6% aqarose (Sigma, St. Louis, MO.) in 10% female FCS supplemented α-MEM+. The overlayer consisted of 0.3% agarose in 10% female FCS supplemented α-MEM+, the cells to be tested (50,000 cells/ml for A431, HT-3, and 0M431; 25,000 cells/ml for HEC-1-A, NIH:OVCAR-3, and Hep 3B), epidersal growth factor (EGF) 10 ng/ml (Sigma, St. Louis, MO.) and one of the following: (a) IAP-MIS (final concentration 30 nM); (b) IAP-salt (final protein concentration 19.3 μg/ml); or (c) vehicle buffer as a negative control. The dishes were incubated in humid air with 5% $CO_2$ at 37°C for 10-21 days. Colonies with more than 30 cells were counted with an inverted microscope (Nikon). The results were expressed as percent survival relative to a control group (number of colonies in the test group x 100/number of colonies in the control group).

E. Liquid Medium Colony Inhibition Assay.

Single cell suspensions were placed and grown in 24-well culture plates (Falcon, Oxnard, CA.,#3047). After cell attachment, only those with good single cell dispersion without clumping were used for further study. Agents to be tested were added in a volume less than 1/10 of the total volume in a well, and were tested in triplicate. The cells were incubated in humid air with 5% $CO_2$ at 37°C. Colonies which formed in 5-7 days were stained with Giemsa solution and those with more than 30 cells were counted with an inverted microscope or by a computer based image analyzer.

F. Comparison of IAP-MIS and IAP-salt on Colony Formation.

IAP-MIS (final concentration 30 mM) and IAP-salt (final protein concentration 19.3 μg/ml) were tested in the liquid medium colony inhibition assay using 0.3 ml per well of A431 (25,000 cells/ml), OM431 (25,000 cells/ml), HT-3 (5000 cells/ml) and RT4 (5000 cells/ml). EGF 50 ng/ml was added to A431 and OM431 to suppress monolayer growth but stimulate colony formation according to the method of Lee (Lee, K, et al. Exp. Cell Res. 173:156-162 (1987)). The result was expressed as percent survival relative to the vehicle buffer control.

G. Dose Dependent Inhibition of A431 Cells by MIS.

After dilution in vehicle buffer, DG-MIS was tested in final concentrations of 0.9, 1.8, 3.5, and 7.0 nM using A431 cells (25,000 cells/ml, EGF 50 ng/ml) in the liquid medium colony inhibition assay. IAP-MIS was tested in concentrations of 12, 24, 48, and 96 nM. The results were expressed as percent survival relative to the vehicle buffer control.

H. Antibody Absorption of MIS.

MIS monoclonal antibody (6E11) and normal rabbit IgG (Dako Corporation, Denmark Lot 108) were dialyzed into serum-free α-MEM+ before use. Previous experiments showed maximum absorption of MIS activity at MIS:Ab ratio of 1:3. Therefore, 17.4 μg of 6E11 was added to 5.6 μg of DG-MIS. Normal rabbit IgG was diluted with culture medium and added to MIS in the same 1:3 ratio to determine nonspecific absorption. An equivalent amount of protein purified from conditioned medium of untransfected wild type CHO cells served as a negative control when mixed 1:3 with antibody as above. The preparations were mixed at 4°C for 12 hours.

Protein A Sepharose 9CL-4B (Sigma, St. Louis, MO.), after being washed in serum free medium, was added and incubated at 4°C for another 12 hours. The mixtures were centrifuged and the supernatants tested in the liquid medium colony inhibition assay using A431 cells (25,000 cells/ml, EGF 50 mg/ml). Percent survival of each group was calculated by comparing the number of colonies in each group to the wild type negative control.

I. Cotreatment of IAP-MIS and the Salt Eluted Fraction (IAP-salt).

IAP-MIS was mixed with an equal volume of IAP-salt (protein concentration 0.199 mg/ml) to give a final MIS concentration of 50 nM. It was tested using A431 cells (25,000 cells/ml, EGF 50 ng/ml) in the liquid medium colony inhibition assay and compared to IAP-MIS, IAP-salt, and vehicle buffer. Percent survival was calculated by comparing the number of colonies in each group to that of vehicle buffer negative control.

J. Cotreatment of MIS and Cisplatin.

Cisplatin (Bristol Laboratory, Syracuse, NY) was diluted in serum free culture medium to give concentrations of 0, 0.078, 0.156, 0.312 and 0.624 μg/ml and tested in triplicate with or without DG-MIS (final concentration 7 nM) using A431 cells (25,000 cells/ml, EGF 50 ng/ml) in the liquid medium colony inhibition assay. Vehicle buffer was used as negative control. The percent survival was calculated by comparing the number of colonies grown at each dose to that achieved in the vehicle buffer negative control.

K. Multicellular Tumor Spheroids Assay.

Multicellular tumor spheroids of HT-3 and Hep 3B cells were produced by the method described by Yuhas et al. (Yuhas, J.M., et al., Cancer Res. 37:3639-3643 (1977)). In brief, either $10^6$ cells of HT-3 or Hep 3B in 10 ml of 10% female FCS supplemented α-MEM+, were plated on the top of 1% agarose in a 10 cm culture dish and incubated in humid air with 5% $CO_2$ at 37°C. Spheroids usually formed in 2-5 days. 0.5 ml of 1% agarose was added to each well of a 24-well culture plate (Falcon, Oxnard, CA.,#3047) to form a bottom layer before use. Individual spheroids of similar size (approximately 250 μm diameter) were selected under a dissecting microscope and transferred by a micropipette at one spheroid per well containing 0.5 ml of 10% female FCS supplemented α-MEM+ on top of the agarose layer. The sizes of the spheroid on day 0 were measured by the longest diameter (L) and the diameter perpendicular to the longest one (W). Volume was then expressed as ( LxWxW ). Six spheroid containing wells of each cell type were treated either by DG-MIS (final concentration 7 nM) or vehicle buffer. Volumes were measured again at the 3rd, 6th and 9th day. The size ratio of each spheroid at different intervals was obtained by comparing it to the size of the same spheroid at day 0. The average size ratio of each group was plotted vs. time in a growth curve and compared to the other groups.

L. Subrenal Capsule Assay.

Following the method of Bogden (Bogden, A.Z., et al., Exp. Cell Biol. 47:281-293 (1979)), which was later modified by Fingert (Fingert, H.J., et al., Cancer Res. 47:3824-3829 (1987)), A431 and OM431 cells were tested. $10^7$ of the cells were centrifuged at 1500 RPM for 5 minutes to form a pellet. 15 μl of fibrinogen (Sigma, St. Louis, MO., 20 mg/ml dissolved in PBS pH 7.4) was added to the pellet, followed by 8 μl of thrombin (Sigma, St. Louis, MO., 20 unit/ml dissolved in double-strength Dulbecco's modified essential medium). The mixture was incubated at 37°C for 15 min. The cell clot thus formed was cut into approximately 100 fragments (1 $mm^3$ each containing approximately $10^5$ cells) in preparation for implantation.

MIS was delivered by an Alzet mini-osmotic pump (#2001, Alza, Palo Alto, CA.) placed in the peritoneal cavity at the time of tumor implantation. These pumps have a fill volume of 209±6 μl and release their contents at a rate of 1.03±0.04 μl/hr for approximately eight days of delivery time. The pumps were either filled with IAP-MIS (MIS: 159 μg/ml by ELISA) or with vehicle buffer. A total MIS dose of approximately 33 μg (230 nM) was given to each mouse in the MIS group over the course of the experiment.

Virus and pathogen free female CD-1 mice (10 weeks old, average weight 35 g, Charles River Breeding Laboratory, Wilmington, MA.) were given whole body irradiation of 640 rads by a Mark-1 cesium-137 irradiation 16-24 hours before the experiment (Gajewski, W.H., et al., Surgical Forum 38:468-470 (1987)). After inducing anesthesia with an intraperitoneal injection of 0.3 ml of 10% Pentobarbital (Abbott Laboratory, North Chicago, IL), an incision was made in the left flank of the mouse and the left kidney exteriorized. A subcapsular space was developed using a 19-gauge needle trocar. A cell clot was introduced into the space with a segment of 5-0 Nylon suture (approximately 1 mm in length), which was used both to calibrate ocular micrometer measurements and to localize the tumor. Twenty-four mice were implanted with A431 cell clots and 12 mice with OM431 cell clots. The longest diameter (LI) of the implant, the one perpendicular to the longest one (W1), and the length of the suture were measured with the ocular micrometer of a dissecting microscope. The animals were either treated by IAP-MIS or vehicle buffer delivered by the Alzet pumps placed in the peritoneal cavity. Blood samples at 6, 24, 48, 120 (fifth day) and 192 hours (eighth day) were obtained from selected animals by orbital bleeding and serum MIS levels were measured by ELISA. The animals were sacrificed on the eighth day. The longest diameter (L1) of the tumor, the one perpendicular to the longest one (W1), and the length of the suture were measured blindly by two independent investigators. After calibration of the measurements, the graft size ratio was represented by (L2 x W1 x W2 / (L1 x W1 x W1 ). Histologic sections of the kidneys were also obtained and examined. Tumors with cystic change were excluded.

M. Statistical Analysis.

The results of the liquid medium and semisolid medium colony inhibition assays were analyzed by the Chi square test, with or without Yates correction, while the multicellular spheroid and the subrenal capsule assays were tested by the Student t-test. $p < 0.05$ was considered as statistically significant.

II. RESULTS

A. Semisolid Medium (Double Layer) Colony Inhibition Assay.

The percent survival of the various cell lines after incubation with 30 nM of IAP-MIS was 45% for A431, 747% for HT-3 , 54% for HEC-I-A, 59% for NIH:OVCAR-3, 34% for OM431, and 114% for Hep 3B. When compared to controls, the survival after treatment with MIS in all cell lines except Hep 3B were significantly inhibited by IAP-MIS ($p < 0.05$). The growth of Hep 3B was not inhibited by MIS. The percent survival after incubation with IAP-salt were 172% for A431, 93% for HT3, 92% for HEC-1-A, 120% for OM431, 105% for NIH:OVCAR-3 and 173% for Hep 133B. The stimulatory effect was significant for A431, OM431 and Hep 3B cells ($p < 0.05$) (FIG. 1).

B. Effect of IAP-MIS and IAP-salt on Colony Formation using the Liquid Medium Colony Inhibition Assay.

The percent survival was 55.9% for A431, 36.8% for OM431, 100.5% for HT-3, and 115.8% for RT4 when treated with 30 nM of IAP-MIS. The percent survival was 169.0% for A431, 226.7% for OM431, 101.6% for HT-3 and 96.5% for RT4 when treated with IAP-salt (final protein concentration 19.3 μg/ml). The inhibitory effect of IAP-MIS and the stimulatory effect of IAP-salt were significant for A431 and OM431 colony formation ($p < 0.05$) (FIG. 2).

C. Dose Dependent Inhibition of A431 Colony Formation by MIS.

The percent survival was 103.4%, 81.4%, 61.3% and 26.5% respectively, for DG-MIS concentrations of 0.9, 1.8, 3.5, and 7.0 nM. Significant inhibitions were seen with DG-MIS concentrations of 3.5 and 7.0 nM ($p < 0.05$). The percent survival were 109.7%, 71.1%, 56.6%, and 33.3% respectively for IAP-MIS concentrations of 12, 24, 48, and 96 nM. Significant inhibitions were seen at IAP-MIS concentrations of 24, 48, and 96 nM (FIG. 3). DG-MIS thus was 10-14 times more potent than IAP-MIS.

D. Antibody Absorption of MIS.

The MIS levels, measured by ELISA for MIS, were 9.0 nM for the positive control (MIS alone), 8.8 nM after normal IgG absorption (nonspecific) and 0.62 nM after the monoclonal antibody absorption (specific). All the wild type negative controls had an MIS level equal to 0. The percent survival was 42.9% for MIS alone, 38.0% for normal IgG (nonspecific absorption), and 74.2% for the monoclonal antibody (specific absorption). There was no significant nonspecific absorption of MIS activity ($p > 0.05$ compared with negative control). The percent survival after the monoclonal antibody absorption was significantly higher than that of positive control (MIS alone) and normal IgG (FIG. 4).

E. The Salt Eluted Fraction (IAP-salt) Inhibits IAP-MIS.

The percent survival of A431 cells was 51.3% for IAP-MIS alone (50 nM), 116.9% for IAP-salt alone and 81.6% for the combination of these two (MIS 50 nM). The difference between IAP-MIS alone and the combination of IAP-MIS/IAP-salt was significant ($p < 0.05$) (FIG. 5). SDS gel electrophoresis was performed after reduction of IAP-salt and two separate preparations of IAP-MIS. Several bands of protein in the IAP-salt were seen in the region of 14-43 kDa, but were absent in the IAP-MIS.

F. Additive Effect of Cisplatin and MIS.

The percent survival of A431 cells was 100%, 56.5%, 38.7%, 34.3% and 10.8% respectively for cisplatin concentrations of 0, 0.078, 0.156, 0.312 and 0.624 µg/ml. With the addition of 7 nM DG-MIS, the percent survival became 39.3%, 32.2%, 23.1%, 20.4% and 5.4%, respectively, for the same concentrations of cisplatin. The percent survival of cisplatin alone and cisplatin plus MIS was significantly different at the cisplatin concentrations of 0.078 and 0.156 µg/ml (FIG. 6).

G. Multicellular Spheroid Assay.

The average size ratios of HT-3 spheroids in the control group (n=6) were 1.15±0.04, 1.44±0.02 and 151.96±0.11 at day 3, 6 and 9 respectively, while in the MIS group (n=6) they were 0.98±0.04, 0.94±0.04 and 1.08±0.06 (FIG. 7A). The average size ratios of Hep 3B spheroids in the control group (n=6) were 2.56±0.05, 5.64±0.53 and 13.07±1.09 respectively at day 3, 6 and 9, while in the MIS group (n=6), they were 2.36±0.25, 5.77±0.54 and 14.30±0.54. The growth of Hep 3B spheroids was faster and uninhibited by MIS (FIG. 7B).

H. Subrenal Capsule Assay.

The MIS levels in souse serum were relatively stable from 24 hours to the eighth day after the implantation of the MIS filled Alzet pumps (FIG. 8A). The average MIS level of 14 blood samples from day 2 to day 8 was 19.1±2.7 ng/ml (approximately 140 pM). The serum MIS levels of the control mice were undetectable. The graft size ratio of the implanted A431 tumor was 3.70±0.31 in the control group (n=12) vs 1.50±0.26 in the MIS group (n=12). The graft size ratio of the OM431 tumor was 3.93±0.49 in the control (n=7) vs 1.42±0.44 in the MIS group (n=5). The growth of the tumors in the MIS group was significantly lower than the control in both cell lines (p<0.05) (FIG. 8B).

III. DISCUSSION

Recombinant human MIS (rhMIS) from the conditioned media of the amplified CHO cell line, purified by either serial ion exchange and dye affinity chromatography (DG-MIS) or immunoaffinity chromatography (IAP-MIS), was examined against various cell lines in a number of growth assays. Overall, the results of the liquid and semisolid assays were quite similar, with the exception of the HT-3 (cervical carcinoma) cell line which was inhibited by highly purified MIS in the semisolid assay but not in the liquid assay. The multicellular spheroid assay was used to recapitulate tumor microregions with cell-cell interactions and nutrient affected growth patterns. HT-3 cells formed satisfactory spheroids that were inhibited by MIS. No MIS effect was noted on the spheroid growth of Hep 3B (hepatocellular carcinoma). The availability of all three in vitro assays permits selection of optimal conditions for each tumor.

In evaluating MIS as an anticancer agent, it is important to consider its interaction with available chemotherapies. To evaluate this, MIS and cisplatin were tested alone and in various combinations. Since the inhibitory effects of MIS and cisplatin are additive, cisplatin doses could be lowered when given with MIS; therefore this biological modifier might function as an adjuvant in the multimodality treatment of selected human malignancies. The liquid colony inhibition and the spheroid assays can be used to test repeated doses of either MIS or MIS plus cytotoxic agents. However, asynchronous addition, first of MIS, which best effects a proliferative cell population, then the cytotoxic agent, is preferable.

DG-MIS, although less purified, consistently showed inhibition of cell growth in vitro. The specificity of the MIS effect was demonstrated by blocking the inhibitory effect with an MIS monoclonal antibody.

Wallen et al. (Wallen, J.W., et al., Cancer Res. 49:2005-2011 (1986)) reported only a minimal antiproliferative activity when an immunopurified MIS preparation was tested against a variety of established cell lines. In Example 1, the same poor response with IAP-MIS was observed before the salt elution step was added prior to elution with 1 M acetic acid. In addition, the salt fraction eluted from the immunoaffinity column actually showed a growth stimulating effect in some cell lines. Electrophoresis of this salt fraction showed several bands in the region of 14-20 kDa, which are absent in the fraction subsequently eluted by 1 M acetic acid. When the concentrated culture medium of untransfected wild-type CHO cells was subjected to the same purification process, the salt fraction, which also showed a stimulatory effect on A431 cells (FIGS. 1 and 2), revealed the presence of the same bands at 14-43 kDa, implying that these protein products of the untransfected CHO cells might act as growth stimulating factors to mask MIS effect. This finding is supported by the results shown in FIG. 5. When the dose responses to highly purified IAP-MIS and DG-MIS were compared, DG-MIS was shown to be 10-fold more potent than the more highly purified IAP-MIS.

The subrenal capsule assay was used to test the effect of MIS in vivo. By delivering MIS via an intraperitoneal constant infusion Alzet pump inserted at the time of tumor implantation, the growth of a vulvar epidermoid carcinoma cell line, A431, and an ocular melanoma line, OM431, were inhibited in vivo. In evaluating this assay, it is important that histology be documented for each different tumor, and the experiment completed before central necrosis occurs. Thus the assay was terminated on day 8, since longer duration led to variable cystic change. Such changes can vary the graft size ratio enough to make comparisons unreliable due to imbibition of fluid and cystic changes. By this careful analysis, the post implantation characteristics of each tumor cell line can be established and artifacts avoided. The in vivo effect in this subrenal assay is achieved at physiologic or picomolar concentrations. In vitro studies, on the other hand, require 50 to 500 fold higher levels, which may reflect failure of activation. No obvious toxicity to animals was observed.

EXAMPLE 2

I. Materials and Methods

A. Production and Purification of rhMIS.

After cloning MIS cDNA and genomic DNA, dihydrofolate reductase deficient CHO cells were cotransfected with a linear construct of both the human MIS and the dihydrofolate reductase genes as in Example 1. The transfected CHO cells were amplified in Methotrexate and grown at 37°C in alpha minimal essential medium without ribonucleosides and deoxyribonucleosides, supplemented with 10% bovine MIS-free female fetal calf serum (FCS). Immunoaffinity chromatography using an anti-human MIS monoclonal antibody was used to purify the rhMIS (90-95% pure) as in Example 1.

The biological activity of MIS was detected in vitro using the rat Müllerian duct regression organ culture assay as in Example 1. MIS concentrations were estimated using an enzyme-linked immunosorbent assay (ELISA) for MIS, and protein concentrations were measured as in Example 1.

B. Cell Lines.

The human ocular melanoma cell lines, OM431, OM464, OM467, and OM482, were established in 1984 and kept in liquid nitrogen until early passage ampules were thawed for this study. They were maintained in the alpha modification of Eagle's medium supplemented with ribonucleosides and deoxyribonucleosides ($\alpha$-MEM+) to which was added 10% female FCS and 1 gm/l L-glutamine. Before study, cells were serially subcultured, then trypsinized at 70-80% confluency. This proliferating cell population was then centrifuged at 1500 RPM for 5 minutes and resuspended with 10% female FCS supplemented medium. Cells were counted in a hemocytometer.

C. Semisolid Medium (Double Layer) Colony Inhibition Assay.

The effect of rhMIS was tested using the conventional double layer agarose colony inhibition assay as in Example 1. The underlayer of the 35 mm culture dishes contained 1 ml of 0.6% agarose (Sigma, St. Louis, MO.) in 10% female FCS supplemented $\alpha$-MEM+. The overlayer consisted of 0.3% agarose in 10% female FCS supplemented a-MEM+, the cells to be tested (50,000 cells/ml for OM431; 25,000 cells/ml for OM464, OM467 and OM482), epidermal growth factor (EGF) 10 ng/ml (Sigma, St. Louis, MO.) and either rhMIS or vehicle buffer as a negative control. The dishes were incubated in humid air with 5% $CO_2$ at 37°C for 10-21 days. Colonies with more than 30 cells were counted with an inverted microscope (Nikon). The results are expressed as percent survival relative to a control group (number of colonies in the test group x 100/number of colonies in the control group ).

D. Liquid Medium Colony Inhibition Assay.

Single cell suspensions of OM431 were placed and grown in 24-well culture plate. (Falcon, Oxnard, CA.,#3047) at a concentration of 8250 cells per well in 0.5 ml media ($\alpha$-MEM+ with 10% female FCS and 50 ng/ml EGF) as in Example 1. After cell attachment, only those with good single cell dispersion without clumping were used for further study. Agents to be tested were added (50 microliters per well) and were tested in triplicate. The cells were incubated in humid air with 5% $CO_2$ at 37°C. Colonies which formed in 5-7 days were stained with Giemsa solution and those with more than 30 cells were counted by eye with an inverted microscope or the counting was automated using a computer based image analyzer.

E. Dose Dependent Inhibition of OM431 Cells by MIS.

After dilution in vehicle buffer, MIS was tested in concentrations of 0.98, 9.8, 25.2, 50.4, 75.6, and 100.8 nM. The results were expressed as percent survival relative to the vehicle buffer control.

F. Multicellular Tumor Spheroids Assay.

Multicellular tumor spheroids of OM467 and OM482 cells were produced as described in Example 1. In brief, $10^5$ cells of OM467 in 1 ml of 10% female FCS supplemented $\alpha$-MEM+, were plated on top of 1.5 ml of 1% agarose in a 35x10 culture dish after thorough washing to remove residual trypsin, and incubated in humid air with 5% $CO_2$ at 37°C for 2-5 days when spheroids usually formed. 0.5 ml of 1% agarose was then added to each well of a 24-well culture plate (Falcon, Oxnard, CA.,#3047). Individual spheroids of similar size (approximately 250 mm diameter) were selected under a dissecting microscope and transferred by micropipette each to a veil containing 0.5 ml of 10% female FCS supplemented $\alpha$-MEM+ on top of the agarose layer. The sizes of the spheroids on day 0 were measured by the longest diameter (L) and the diameter perpendicular to the longest one (W) and volume then expressed as (LxWxW ). Six spheroid containing wells were treated either by rhMIS or vehicle buffer. Volumes were measured at regular interval. The size ratio of each spheroid at different intervals was obtained by comparing it to the size of the same spheroid at day 0. The average size ratio of each group was plotted vs. time in a growth curve and compared to the other groups.

G. Subrenal Capsule Assay.

Following the method described in Example 1, OM431 cells grew to graft size of $3.68 \pm 0.56$; OM482 to $1.97 \pm 0.67$; OM467 to $1.34 \pm 0.5$; and OM464 <1. Thereafter, $10^7$ OM431 cells were centrifuged at 1500 RPM for 5 minutes to form a pellet. 20 ml of fibrinogen (Sigma, St. Louis, MO., 20 mg/ml dissolved in PBS pH 7.4) was added to the pellet, followed by 10 ml of thrombin (Sigma, St. Louis, MO., 20 unit/ml dissolved in double-strength Dulbecco's modified essential medium). The mixture was incubated at 37°C for 15 min. The cell clot thus formed was cut into approximately 50 fragments (1 $mm^3$, each containing approximately $10^5$ cells) in preparation for implantation.

MIS was delivered by an Alzet mini-osmotic pump (#2001, Alza, Palo Alto, CA.) placed in the peritoneal cavity at the time of tumor implantation. These pumps have a fill volume of approximately 210 µl and release their contents at a rate of approximately 1 µl/hr for eight days of delivery time. The pumps were either filled with rhMIS or with vehicle buffer.

Virus and pathogen free female CD-1 mice (10 weeks old, average weight 35 g, Charles River Breeding Laboratory, Wilmington, MA.) were given whole body irradiation of 640 rads by a Mark-I cesium-137 irradiator 16-24 hours before the experiment as in Example 1. Nude mice (8 weeks old, average weight 24 g, Edwin L. Steele Laboratory, Massachusetts General Hospital, Boston, HA) were also used. After inducing anesthesia with an intraperitoneal injection of 0.3 ml of 10% Pentobarbital (Abbott Laboratory, North Chicago, IL), an incision was made in the left flank of the mouse and the left kidney exteriorized. A subcapsular space was developed using a 19-gauge needle trocar. A cell clot was introduced into the space with a sequent of 5-0 Nylon suture (approximately 1 mm in length), which was used both to calibrate ocular micrometer measurements and to localize the tumor. Twelve CD-1 mice and twenty nude mice were implanted with OM431 cell clots. The longest diameter (L1) of the implant, the diameter perpendicular to the longest diameter (W1), and the length of the suture were measured with the ocular micrometer of a dissecting microscope. The animals were either treated by rhMIS or vehicle buffer delivered by the Alzet pumps placed in the peritoneal cavity. The animals were sacrificed on the eighth day. Blood samples were obtained on the eighth day from the nude mice and serum MIS levels were measured by ELISA. The longest diameter (L2) of the tumor, the diameter perpendicular to the longest diameter (W2), and the length of the suture were measured blindly by two independent investigators. After calibration of the measurements, the graft size ratio was represented by (L2 x W2 x W2) / (L1 x W1 x W1) . Histologic sections of the kidneys were also obtained and examined. Tumors with cystic change were excluded.

H. Statistical Analysis.

The results of the liquid medium, semisolid medium colony inhibition, multicellular spheroid and subrenal capsule assays were tested by the Student t-test. $P<0.05$ was considered as statistically significant.

II. Results

A. Dose Dependent Inhibition of OM431 Colony Formation by MIS in the Liquid Colony Inhibition Assay.

Only OM431 cells achieved adequate growth with its ligand colony inhibition assay. The percent survival was 33%, 29.5%, 56.6%, 71%, 109.8%, and 96.8% respectively for MIS concentrations of 100.8, 75.6, 50.4, 25.2, 9.8, and 0.98 nM. Significant inhibitions were seen with MIS concentrations of 50.4 (p<.05), 75.6 (p<.004), and 100.8 (p<.006) nM MIS (FIG. 9).

B. Semisolid Medium (Double Layer) Colony Inhibition Assay.

All cell lines, except OM434 which failed to grow colonies, were significantly inhibited by rhMIS. The percent survival of the various cell lines was 34% for OM431 (in 30 nM MIS) (p<.01), 61% for OM467 (in 150 nM MIS) (p<.02), and 80% for OM482 (in 90 nM MIS) (p<.03) (FIG. 2).

C. Multicellular Spheroid Assay.

The average size ratios of OM 467 spheroids in the control group (n=6) were 3.85±0.43, 4.85±0.64 and 5.52±0.78 at day 5, 8 and 11, respectively, while in the MIS group (n=6) they were 2.17±0.26, 2.79±0.33 and 3.91±0.59. This difference was significant (p<.02). The average size ratio of OM482 spheroids in the control group (n=10) were 2.18±0.17 and 8.22±0.81 respectively at day 3 and 6, while in the MIS group (n=10), they were 1.93±0.14 and 9.0±0.67 (FIGS. 11A & B). OM431 and OM464 failed to grow as spheroids.

D. Subrenal Capsule Assay.

In the CD-1 irradiated nice, the graft size ratio of the OM431 tumor was 3.93±0.49 in the control (n=7) vs 1.42±0.44 (p<.005) in the MIS group (n=5). Each MIS treated mouse received 48.6 micrograms of MIS over the eight day assay. In two assays using nude mice, with eleven controls and nine MIS treated mice, the graft size ratios of the OM431 tumors were significantly greater, 3.02±0.17 and 3.14±0.40, respectively, for the controls, vs 1.68±0.09 (p<.001) and 1.69±0.43 (p<.005) for the MIS group. In the first nude mouse assay, the MIS treated group received 44.7 micrograms MIS over eight days while in the second nude mouse assay, the MIS group received 130 micrograms. The average MIS serum levels of the MIS treated mice on the eighth day of the nude mouse assays were 749 and 570 pM respectively. The measured controls had MIS levels of less than 10 pM. The growth of the tumors in each MIS group was significantly lower than the control in all three assays (p<0.05) (FIGS. 12A-C).

III. Discussion

In Example 2, the effect of recombinant human MIS on three human ocular melanoma cell lines using three different in vitro clonogenic assays were examined. The reliable and reproducible double-layer inhibition assay was used with each cell line. Despite its rather lengthy incubation time (10-21 days), all the cell lines grew well in this assay. OM482, OM43 1 and OM467 were significantly inhibited. The liquid colony inhibition assay was used with the OM431 cell line. OM467 and OM482 failed to grow discrete colonies in this assay. When effective, as with OM431, this assay is rapid (5-7 days) and uses little sample. OM431 showed a clear dose response with inhibition of colony formation with rhMIS concentrations above 25 nM. The percent survival of OM431 correlated well between the two assays. The multicellular spheroid assay was used to recapitulate tumor microregions with cell-cell interactions and nutrient affected growth patterns. OM467 and 482 cell lines formed satisfactory spheroids. OM467 showed significant inhibition while the growth of OM482 was unaffected. OM431 failed to grow spheroids. The availability of all three in vitro assays permits selection of optimal conditions for each tumor.

The subrenal capsule assay was used to test the effect of MIS in vivo against OM431, since this line grew large enough to permit comparisons. By delivering MIS via an intraperitoneal constant infusion Alzet pump inserted at the time of tumor implantation, the growth of OM431 was inhibited in vivo. It is important in evaluating this assay that histology be documented for each different tumor, and the experiment completed while the tumors are still solid and before a lymphocytic infiltrate and/or central necrosis occurs. Thus, the assay was terminated on day 8 in the irradiated CD-1 mice since a longer duration of incubation led to variable histologic changes which can vary the graft size ratio enough to make comparisons unreliable due to inhibition of short lived fluid, inflammatory cell infiltration and cystic changes. To eliminate the potential pitfalls of radiation induced immunosuppression, nude mice were also used and similar tumor growth inhibition was seen. The same period of time was used when tumors were implanted in the nude mice. No obvious toxicity to animals was observed during the short course of this study. The in vivo effect in this subrenal assay is achieved at picomolar concentrations. In vitro studies, on the other hand, require 10 to 100 fold higher levels, suggesting a failure of cleavage and activation.

**Claims**

**Claims for the following Contracting States : AT, BE, CH, DE, DK, FR, GB, IT, LI, LU, MC, NL, SE**

1. A method of purifying MIS comprising:

   (a) binding the MIS to an antibody-chromatography matrix, said antibody being specific to MIS;
   (b) substantially removing contaminating enzymes having MIS proteolytic activity or inhibitors of MIS antiproliferative activity by adding to the matrix an effective amount of an alkali metal halide or an alkaline earth metal halide; and
   (c) recovering the MIS by eluting with an acid solution having a pH of between about 2.5 and 4.0.

2. A method as claimed in claim 1, wherein the MIS is recombinant MIS.

3. A method as claimed in claim 1 or claim 2, further comprising adding an effective amount of a chelating agent with the halide of step (b).

4. A method as claimed in any one of claims 1 to 3, further comprising neutralising the recovered MIS to a pH of between about 6.8 and 7.6

5. A method as claimed in any one of claims 1 to 4, wherein the alkali metal halide solution is between about 0.1 M and 2.0 M.

6. A method as claimed in any one of claims 1 to 5, wherein the alkali metal halide is sodium chloride.

7. A method as claimed in any one of claims 1 to 6, wherein the acid is acetic acid.

8. The use of recombinant MIS in the preparation of a pharmaceutical agent for inhibiting growth of a tumour selected from the group consisting of vulvar epidermoid carcinoma, cervical carcinoma, endometrial adenocarcinoma, ovarian adenocarcinoma and ocular melanoma, wherein said MIS is obtainable by a purification method of any one of claims 1 to 7 and is substantially free of anti-proliferative inhibitors.

9. The use of recombinant MIS of claim 8, wherein said MIS has a molecular weight of 140 kDa or 70 kDa.

10. The use of recombinant MIS of claim 8, wherein said MIS is proteolytically cleaved by reacting with a proteolytic compound to form protein fragments having a molecular weight of about 57 kDa and 12.5 kDa.

11. The use of recombinant MIS according to claim 10, wherein said proteolytic compound is plasmin.

12. The use of recombinant MIS of any one of claims 8 to 10, further comprising adding an effective amount of a chemotherapeutic agent in the preparation of said pharmaceutical agent, said chemotherapeutic agent effective in inhibiting growth of said tumour.

13. The use of claim 12, wherein said chemotherapeutic agent is cisplatin.

14. A process for the preparation of a composition comprising admixing recombinant MIS which is substantially free of anti-proliferative inhibitors obtainable by a purification method as claimed in any one of claims 1 to 7 and a pharmaceutically acceptable carrier.

15. A composition comprising recombinant MIS substantially free of anti-proliferative inhibitors and obtainable by the purification method of any one of claims 1 to 7.

**Claims for the following Contracting States : ES, GR**

1. A method of purifying MIS comprising:

   (a) binding the MIS to an antibody-chromatography matrix, said antibody being specific to MIS;
   (b) substantially removing contaminating enzymes having MIS proteolytic activity or inhibitors of MIS antiproliferative activity by adding to the matrix an effective amount of an alkali metal halide or an alkaline earth metal halide; and

(c) recovering the MIS by eluting with an acid solution having a pH of between about 2.5 and 4.0.

**2.** A method as claimed in claim 1, wherein the MIS is recombinant MIS.

**3.** A method as claimed in claim 1 or claim 2, further comprising adding an effective amount of a chelating agent with the halide of step (b).

**4.** A method as claimed in any one of claims 1 to 3, further comprising neutralising the recovered MIS to a pH of between about 6.8 and 7.6

**5.** A method as claimed in any one of claims 1 to 4, wherein the alkali metal halide solution is between about 0.1 M and 2.0 M.

**6.** A method as claimed in any one of claims 1 to 5, wherein the alkali metal halide is sodium chloride.

**7.** A method as claimed in any one of claims 1 to 6, wherein the acid is acetic acid.

**8.** The use of recombinant MIS in the preparation of a pharmaceutical agent for inhibiting growth of a tumour selected from the group consisting of vulvar epidermoid carcinoma, cervical carcinoma, endometrial adenocarcinoma, ovarian adenocarcinoma and ocular melanoma, wherein said MIS is obtainable by a purification method of any one of claims 1 to 7 and is substantially free of anti-proliferative inhibitors.

**9.** The use of recombinant MIS of claim 8, wherein said MIS has a molecular weight of 140 kDa or 70 kDa.

**10.** The use of recombinant MIS of claim 8, wherein said MIS is proteolytically cleaved by reacting with a proteolytic compound to form protein fragments having a molecular weight of about 57 kDa and 12.5 kDa.

**11.** The use of recombinant MIS according to claim 10, wherein said proteolytic compound is plasmin.

**12.** The use of recombinant MIS of any one of claims 8 to 10, further comprising adding an effective amount of a chemotherapeutic agent in the preparation of said pharmaceutical agent, said chemotherapeutic agent effective in inhibiting growth of said tumour.

**13.** The use of claim 12, wherein said chemotherapeutic agent is cisplatin.

**14.** A process for the preparation of a composition comprising admixing recombinant MIS which is substantially free of anti-proliferative inhibitors obtainable by a purification method as claimed in any one of claims 1 to 7 and a pharmaceutically acceptable carrier.

**Patentansprüche**

**Patentsprüche für folgende Vertragsstaaten : AT, BE, CH, DE, DK, FR, GB, IT, LI, LU, MC, NL, SE**

**1.** Verfahren zum Reinigen von MIS, umfassend:

(a) Binden des MIS an eine Antikörper-Chromatographie-Matrix, wobei der Antikörper für MIS spezifisch ist;
(b) in wesentlichem Umfang Entfernen kontaminierender Enzyme mit MIS-proteolytischer Aktivität oder von Inhibitoren der antiproliferativen Aktivität des MIS durch Zugabe einer wirksamen Menge eines Alkalimetallhalogenids oder eines Erdalkalimetallhalogenids zu der Matrix; und
(c) Gewinnen des MIS durch Elution mit einer einen pH-Wert zwischen etwa 2,5 und 4,0 aufweisenden sauren Lösung.

**2.** Verfahren nach Anspruch 1, worin das MIS rekombinantes MIS ist.

**3.** Verfahren nach Anspruch 1 oder 2, das des weiteren die Zugabe einer wirksamen Menge eines chelatisierenden Mittels mit dem Halogenid aus Schritt (b) umfaßt.

4. Verfahren nach mindestens einem der Ansprüche 1 bis 3, das des weiteren das Neutralisieren des gewonnenen MIS auf einen pH-Wert zwischen etwa 6,8 und 7,6 umfaßt.

5. Verfahren nach mindestens einem der Ansprüche 1 bis 4, worin die Alkalimetallhalogenidlösung zwischen etwa 0,1 M und 2,0 M liegt.

6. Verfahren nach mindestens einem der Ansprüche 1 bis 5, worin das Alkalimetallhalogenid Natriumchlorid ist.

7. Verfahren nach mindestens einem der Ansprüche 1 bis 6, worin die Säure Essigsäure ist.

8. Verwendung von rekombinanter MIS bei der Herstellung eines pharmazeutischen Mittels zur Inhibierung des Wachstums eines Tumors, ausgewählt aus der Gruppe, die aus vulvar epidermoidem Karzinom, cervicularem Karzinom, endometrialem Adenokarzinom, ovarem Adenokarzinom und ocularem Melanom besteht, wobei das MIS nach einem Reinigungsverfahren nach mindestens einem der Ansprüche 1 bis 7 erhältlich ist und im wesentlichen frei von Antiproliferativinhibitoren ist.

9. Verwendung von rekombinantem MIS nach Anspruch 8, wobei das MIS ein Molekulargewicht von 140 kDa oder 70 kDa aufweist.

10. Verwendung von rekombinantem MIS nach Anspruch 8, wobei das MIS durch Reaktion mit einer proteolytischen Verbindung proteolytisch gespalten wird, um Proteinfragmente mit einem Molekulargewicht von etwa 57 kDa und 12,5 kDa zu bilden.

11. Verwendung von rekombinantem MIS nach Anspruch 10, wobei die proteolytische Verbindung Plasmin ist.

12. Verwendung von rekombinantem MIS nach mindestens einem der Ansprüche 8 bis 10, die des weiteren die Zugabe einer wirksamen Menge eines chemotherapeutischen Mittels zur Zusammensetzung des pharmazeutischen Mittels umfaßt, wobei das chemotherapeutische Mittel zur Inhibierung des Wachstums des Tumors wirksam ist.

13. Verwendung nach Anspruch 12, wobei das chemotherapeutische Mittel Cisplatin ist.

14. Verfahren zur Herstellung einer Zusammensetzung, umfassend das Mischen von rekcombinantem MIS, das im wesentlichen frei von Antiprolieferativinhibitoren ist, erhältlich durch ein Reinigungsverfahren nach mindestens einem der Ansprüche 1 bis 7, und einem pharmazeutisch akzeptablen Träger.

15. Zusammensetzung, umfassend rekombinantes MIS, das im wesentlichen frei von Antiproliverativinhibitoren ist und nach dein Reinigungsverfahren nach mindestens einem der Ansprüche 1 bis 7 erhältlich ist.

**Patentansprüche für folgende Vertragsstaaten : ES, GR**

1. Verfahren zum Reinigen von MIS, umfassend:

   (a) Binden des MIS an eine Antikörper-Chromatographie-Matrix, wobei der Antikörper für MIS spezifisch ist;
   (b) in wesentlichem Umfang Entfernen kontaminierender Enzyme mit MIS-proteolytischer Aktivität oder von Inhibitoren der antiproliferativen Aktivität des MIS durch Zugabe einer wirksamen Menge eines Alkalimetallhalogenids oder eines Erdalkalimetallhalogenids zu der Matrix; und
   (c) Gewinnen des MIS durch Elution mit einer einen pH-Wert zwischen etwa 2,5 und 4,0 aufweisenden sauren Lösung.

2. Verfahren nach Anspruch 1, worin das MIS rekombinantes MIS ist.

3. Verfahren nach Anspruch 1 oder 2, das des weiteren die Zugabe einer wirksamen Menge eines chelatisierenden Mittels mit dem Halogenid aus Schritt (b) umfaßt.

4. Verfahren nach mindestens einem der Ansprüche 1 bis 3, das des weiteren das Neutralisieren des gewonnenen MIS auf einen pH-Wert zwischen etwa 6,8 und 7,6 umfaßt.

**5.** Verfahren nach mindestens einem der Ansprüche 1 bis 4, worin die Alkalimetallhalogenidlösung zwischen etwa 0,1 M und 2,0 M liegt.

**6.** Verfahren nach mindestens einem der Ansprüche 1 bis 5, worin das Alkalimetallhalogenid Natriumchlorid ist.

**7.** Verfahren nach mindestens einem der Ansprüche 1 bis 6, worin die Säure Essigsäure ist.

**8.** Verwendung von rekombinanter MIS bei der Herstellung eines pharmazeutischen Mittels zur Inhibierung des Wachstums eines Tumors, ausgewählt aus der Gruppe, die aus vulvar epidermoidem Karzinom, cervicularem Karzinom, endometrialem Adenokarzinom, ovarem Adenokarzinom und ocularem Melanom besteht, wobei das MIS nach einem Reinigungsverfahren nach mindestens einem der Ansprüche 1 bis 7 erhältlich ist und im wesentlichen frei von Antiproliferativinhibitoren ist.

**9.** Verwendung von rekombinantem MIS nach Anspruch 8, wobei das MIS ein Molekulargewicht von 140 kDa oder 70 kDa aufweist.

**10.** Verwendung von rekombinantem MIS nach Anspruch 8, wobei das MIS durch Reaktion mit einer proteolytischen Verbindung proteolytisch gespalten wird, um Proteinfragmente mit einem Molekulargewicht von etwa 57 kDa und 12,5 kDa zu bilden.

**11.** Verwendung von rekombinantem MIS nach Anspruch 10, wobei die proteolytische Verbindung Plasmin ist.

**12.** Verwendung von rekombinantem MIS nach mindestens einem der Ansprüche 8 bis 10, die des weiteren die Zugabe einer wirksamen Menge eines chemotherapeutischen Mittels zur Zusammensetzung des pharmazeutischen Mittels umfaßt, wobei das chemotherapeutische Mittel zur Inhibierung des Wachstums des Tumors wirksam ist.

**13.** Verwendung nach Anspruch 12, wobei das chemotherapeutische Mittel Cisplatin ist.

**14.** Verfahren zur Herstellung einer Zusammensetzung, umfassend das Mischen von rekombinantem MIS, das im wesentlichen frei von Antiprolieferativinhibitoren ist, erhältlich durch ein Reinigungsverfahren nach mindestens einem der Ansprüche 1 bis 7, und einem pharmazeutisch akzeptablen Träger.

**Revendications**

**Revendications pour les Etats contractants suivants : AT, BE, CH, DE, DK, FR, GB, IT, LI, LU, MC, NL, SE**

**1.** Procédé pour purifier la MIS comprenant :

(a) la liaison de la MIS à une matrice de chromatographie par anticorps, ledit anticorps étant spécifique à la MIS;
(b) l'élimination substantielle d'enzymes contaminants ayant une activité protéolytique sur la MIS ou d'inhibiteurs de l'activité antiproliférative de la MIS en ajoutant à la matrice une quantité efficace d'un halogénure de métal alcalin ou d'un halogénure de métal alcalino-terreux; et
(c) la récupération de la MIS en éluant avec une solution acide ayant un pH d'entre environ 2,5 et 4,0.

**2.** Procédé tel que revendiqué à la revendication 1, dans lequel la MIS est de la MIS recombinante.

**3.** Procédé tel que revendiqué à la revendication 1 ou à la revendication 2, comprenant en outre l'addition d'une quantité efficace d'un agent chélateur avec l'halogénure de l'étape (b).

**4.** Procédé tel que revendiqué à l'une quelconque des revendications 1 à 3, comprenant en outre la neutralisation de la MIS récupérée à un pH d'entre environ 6,8 et 7,6.

**5.** Procédé tel que revendiqué à l'une quelconque des revendications 1 à 4, dans lequel la solution d'halogénure de métal alcalin est entre environ 0,1 M et 2,0 M.

**6.** Procédé tel que revendiqué à l'une quelconque des revendications 1 à 5, dans lequel l'halogénure de métal alcalin est du chlorure de sodium.

**7.** Procédé tel que revendiqué à l'une quelconque des revendications 1 à 6, dans lequel l'acide est de l'acide acétique.

**8.** Utilisation de la MIS recombinante dans la préparation d'un agent pharmaceutique pour inhiber la croissance d'une tumeur sélectionnée parmi le groupe comprenant un carcinome épidermoide vulvaire, un carcinome cervical, un adénocarcinome d'endomètre, un adénocarcinome d'ovaires et un mélanome oculaire, dans laquelle ladite MIS peut être obtenue par un procédé de purification suivant l'une quelconque des revendications 1 à 7 et est pratiquement exempte d'inhibiteurs d'antiprolifération.

**9.** Utilisation de la MIS recombinante suivant la revendication 8, dans laquelle ladite MIS a un poids moléculaire de 140 kDa ou 70 kDa.

**10.** Utilisation de la MIS recombinante suivant la revendication 8, dans laquelle ladite MIS est clivée de manière protéolytique par réaction avec un composé protéolytique pour former des fragments protéiques ayant un poids moléculaire d'environ 57 kDa et 12,5 kDa.

**11.** Utilisation de la MIS recombinante suivant la revendication 10, dans laquelle ledit composé protéolytique est de la plasmine.

**12.** Utilisation de la MIS recombinante suivant l'une quelconque des revendications 8 à 10, comprenant en outre l'addition d'une quantité efficace d'un agent chimiothérapeutique dans la préparation dudit agent pharmaceutique, ledit agent chimiothérapeutique étant efficace au niveau de l'inhibition de la croissance de ladite tumeur.

**13.** Utilisation suivant la revendication 12, dans laquelle ledit agent chimiothérapeutique est du cisplatine.

**14.** Procédé pour la préparation d'une composition comprenant le mélange additionnel de la MIS recombinante qui est pratiquement exempte d'inhibiteurs d'antiprolifération, pouvant être obtenue par un procédé de purification tel que revendiqué à l'une quelconque des revendications 1 à 7 et un support pharmaceutiquement acceptable.

**15.** Composition comprenant de la MIS recombinante pratiquement exempte d'inhibiteurs d'antiprolifération et pouvant être obtenue par le procédé de purification tel que revendiqué dans l'une quelconque des revendications 1 à 7.

**Revendications pour les Etats contractants suivants : ES, GR**

**1.** Procédé pour purifier la MIS comprenant :

(a) la liaison de la MIS à une matrice de chromatographie par anticorps, ledit anticorps étant spécifique à la MIS;
(b) l'élimination substantielle d'enzymes contaminants ayant une activité protéolytique sur la MIS ou d'inhibiteurs de l'activité antiproliférative de la MIS en ajoutant à la matrice une quantité efficace d'un halogénure de métal alcalin ou d'un halogénure de métal alcalino-terreux; et
(c) la récupération de la MIS en éluant avec une solution acide ayant un pH d'entre environ 2,5 et 4,0.

**2.** Procédé tel que revendiqué à la revendication 1, dans lequel la MIS est de la MIS recombinante.

**3.** Procédé tel que revendiqué à la revendication 1 ou à la revendication 2, comprenant en outre l'addition d'une quantité efficace d'un agent chélateur avec l'halogénure de l'étape (b).

**4.** Procédé tel que revendiqué à l'une quelconque des revendications 1 à 3, comprenant en outre la neutralisation de la MIS récupérée à un pH d'entre environ 6,8 et 7,6.

**5.** Procédé tel que revendiqué à l'une quelconque des revendications 1 à 4, dans lequel la solution d'halogénure de métal alcalin est entre environ 0,1 M et 2,0 M.

**6.** Procédé tel que revendiqué à l'une quelconque des revendications 1 à 5, dans lequel l'halogénure de métal alcalin est du chlorure de sodium.

**7.** Procédé tel que revendiqué à l'une quelconque des revendications 1 à 6, dans lequel l'acide est de l'acide acétique.

**8.** Utilisation de la MIS recombinante dans la préparation d'un agent pharmaceutique pour inhiber la croissance d'une tumeur sélectionnée parmi le groupe comprenant un carcinome épidermoïde vulvaire, un carcinome cervical, un adénocarcinome d'endomètre, un adénocarcinome d'ovaires et un mélanome oculaire, dans laquelle ladite MIS peut être obtenue par un procédé de purification suivant l'une quelconque des revendications 1 à 7 et est pratiquement exempte d'inhibiteurs d'antiprolifération.

**9.** Utilisation de la MIS recombinante suivant la revendication 8, dans laquelle ladite MIS a un poids moléculaire de 140 kDa ou 70 kDa.

**10.** Utilisation de la MIS recombinante suivant la revendication 8, dans laquelle ladite MIS est clivée de manière protéolytique par réaction avec un composé protéolytique pour former des fragments protéiques ayant un poids moléculaire d'environ 57 kDa et 12,5 kDa.

**11.** Utilisation de la MIS recombinante suivant la revendication 10, dans laquelle ledit composé protéolytique est de la plasmine.

**12.** Utilisation de la MIS recombinante suivant l'une quelconque des revendications 8 à 10, comprenant en outre l'addition d'une quantité efficace d'un agent chimiothérapeutique dans la préparation dudit agent pharmaceutique, ledit agent chimiothérapeutique étant efficace au niveau de l'inhibition de la croissance de ladite tumeur.

**13.** Utilisation suivant la revendication 12, dans laquelle ledit agent chimiothérapeutique est du cisplatine.

**14.** Procédé pour la préparation d'une composition comprenant le mélange additionnel de la MIS recombinante qui est pratiquement exempte d'inhibiteurs d'antiprolifération pouvant être obtenue par un procédé de purification tel que revendiqué à l'une quelconque des revendications 1 à 7 et un support pharmaceutiquement acceptable.

## FIG. 1

### Semisolid Medium Colony Inhibition Assay

## FIG. 2

### Liquid Medium Colony Inhibition Assay

# FIG. 3

## Liquid Medium Colony Inhibition Assay

# FIG. 4

## Antibody Absorption of MIS Activity

# FIG. 5

## Inhibition of MIS Effect by IAP-salt

# FIG. 6

## Additive Effect of Cisplatin and MIS

## FIG. 7A

HT-3 Spheroids

□ Control
◆ MIS

n=6

n=6

Size Ratio

day 0   day 3   day 6   day 9

Time

## FIG. 7B

Hep 3B spheroids

n=6
n=6

□ Control
◆ MIS

Size Ratio

day 0   day 3   day 6   day 9

Time

## FIG. 8A

## FIG. 8B

## FIG. 9

## FIG. 10

FIG. 11A

FIG. 11B

FIG. I2A

FIG. I2B

FIG. I2C